# EUROPEAN PATENT APPLICATION

(11) **EP 4 796 090 A1**
(43) Date of publication of application: **26.08.2026**
(21) Application number: 26160088.6
(22) Date of filing: 23.02.2026
(51) Int. Cl.: A61F 13/15, B65B 61/00

(54) **METHOD FOR MANUFACTURING A DISPOSABLE ABSORBENT ARTICLE**

(30) Priority: 24.02.2025 US 202563762108 P
(71) Applicant: The Procter & Gamble Company, Cincinnati, OH 45202 (US)
(72) Inventor: FINDLEY, Daniel Patrick, Cincinnati, 45202 (US); SCHNEIDER, Uwe, Cincinnati, 45202 (US); ECKSTEIN, Joseph Allen, Cincinnati, 45202 (US)
(74) Representative: P&G Patent Germany

(57) **Abstract**

A method of manufacturing a disposable absorbent article is provided. A cylinder of one or more rolls of fibrous material is provided which is partially wrapped with a polymeric covering. A zone of weakness is formed in an axial direction of the cylinder in the polymeric covering using a robot. A breaking device is provided at a temperature within 150 degrees Celsius of the melting temperature of the polymeric covering. Using the robot, a substantially uniform radially inward force is applied against the cylinder with the breaking device along the zone of weakness to rupture the zone of weakness. The method does not damage outer layers of the fibrous material. A robot is used to remove the polymeric covering from the at least one roll. The at least one roll is unwound and fed to the at least one roll into an absorbent article manufacturing line.

## Description

### CROSS REFERENCE TO RELATED APPLICATION

This application claims the benefit, under 35 U.S.C. §119(e), of U.S. Provisional Application No. 63/762,108, filed February 24, 2025, the entire disclosure of which is incorporated herein by reference.

### FIELD

The present disclosure relates generally to disposable absorbent articles and more specifically to a method for manufacturing a disposable absorbent article.

### BACKGROUND

Conventional methods for manufacturing disposable absorbent articles typically utilize fibrous nonwoven material that comes in units of rolls that are packaged together in a cylinder and wrapped in a polymeric covering. Thus, the polymeric covering must first be removed in order to access the individual rolls of fibrous nonwoven material to manufacture the disposable absorbent articles.

Although conventional methods have developed techniques for removing the polymeric covering from the rolls of nonwoven material, these methods typically involve a worker manually operating a cutting tool, such as a utility razor blade or T-handle, to cut and remove the polymeric covering. However, this conventional step has at least two noticeable drawbacks. First, the typical time required to remove the polymeric covering is relatively long, on the order of 1 - 7 minutes and is generally faster when the worker is more careless. Second, manual operation routinely leads to cutting into outer non-woven layers when the worker is not careful. When a worker knows they cannot cut into the outer non-woven layers, there is typically a four-fold increase in the time for operating the cutting tool. In this instance, the time required to unwrap the polymeric covering from the rolls of nonwoven material may increase from about 1.6 minutes to about 6.5 minutes.

The discussion of shortcomings and needs existing in the field prior to the present disclosure is in no way an admission that such shortcomings and needs were recognized by those skilled in the art prior to the present disclosure.

### SUMMARY

Various embodiments solve the above-mentioned problems and provide methods and devices useful for manufacturing disposable absorbent articles including automatically removing a polymeric covering from a cylinder of one or more rolls of fibrous material in a timely efficient manner and with minimal risk of damage to the fibrous material.

It was recognized that since the conventional method for removing the polymeric covering from the cylinder takes a relatively long amount of time (e.g., about 3 minutes), it would be advantageous to develop a method where the polymeric covering was automatically removed from the cylinder in a much shorter amount of time (e.g. about 30 - 45 seconds). Over the course of removal of polymeric covering from hundreds of cylinders, which is typical at many manufacturing plants, this may result in a massive increase in time efficiency of the method.

Additionally, it was recognized that since the conventional step of manually operating a cutting tool to remove the polymeric covering routinely results in damage to one or more rolls such that they cannot be automatically fed to the absorbent article manufacturing line, it would be advantageous to provide a method that minimized damage to the one or more rolls while removing the polymeric covering.

In one set of embodiments, the disclosure is directed to a method of manufacturing a disposable absorbent article. The method may include a step of providing a cylinder of one or more rolls of fibrous material. The cylinder of the one or more rolls of fibrous material may be partially or completely wrapped with a polymeric covering having a tension in the circumferential direction of the cylinder. The method may also include a step of forming a zone of weakness in an axial direction of the cylinder in the polymeric covering using a robot. The zone of weakness includes land areas and open areas. The method may also include a step of providing a breaking device at a temperature within 150 degrees Celsius of the melting temperature of the polymeric covering. The fibrous material may have a melting temperature above the melting temperature of the polymeric covering by at least 10 degrees Celsius. Using the robot, the method may also include a step of applying a substantially uniform radially inward force against the cylinder with the breaking device along the zone of weakness to rupture the zone of weakness. During the applying step, the method may also include not damaging outer layers of the fibrous material. The method may also include a step of using a robot to remove the polymeric covering from the at least one roll. The method may also include a step of unwinding the at least one roll and feeding the at least one roll into an absorbent article manufacturing line.

These and other features, aspects, and advantages of various embodiments will become better understood with reference to the following description, figures, and claims.

### BRIEF DESCRIPTION OF THE DRAWINGS

Many aspects of this disclosure can be better understood with reference to the following figures, which illustrate examples according to various embodiments.
FIG. 1 is a block diagram of an example of a system for manufacturing a disposable absorbent article;
FIGS. 2A through 2C are various views of an example of the system of FIG. 1 being used to remove a polymeric covering from a cylinder including a plurality of rolls of fibrous material;
FIGS. 3A through 3D are various views of an example of an end of a robot arm of the system of FIG. 2A;
FIGS. 4A through 4D are various views of an example of a breaking device and a perforating device at the end of the robot arm of FIGS. 3A through 3D;
FIG. 4E is a side view of an example of a breaking device and a perforating device at the end of the robot arm of FIGS. 3A through 3D:
FIG. 5A is a side view of an example of the perforating device forming a line of weakness in the polymeric covering and a breaking device rupturing the polymeric covering along the line of weakness;
FIG. 5B is cross-sectional view of an example of a roll in the cylinder showing multiple outer layers of the roll fused together over a fused width portion of the roll width;
FIGS. 6A through 6E are perspective views of an example of various stages of a method for automatically removing the polymeric covering from the cylinder using the system of FIG. 1;
FIG. 6F is a perspective view of an example of the separated polymeric covering from the cylinder after performing the stages of the method in FIGS. 6A through 6E;
FIG. 7 is perspective view of a cylinder of rolls wrapped in the polymeric covering and with a sacrificial material positioned intermediate the polymeric covering and the rolls;
FIG. 8 is a flowchart depicting an example of one or more steps of a method for automatically removing a polymeric covering from a cylinder including one or more roll of fibrous material
FIG. 9 is a block diagram that illustrates a computer system upon which an aspect of the disclosure may be implemented;
FIG. 10 is a block diagram that illustrates a chip set upon which an aspect of the disclosure may be implemented; and
FIG. 11 is a block diagram that illustrates a mobile terminal upon which an aspect of the disclosure may be implemented.

It should be understood that the various embodiments are not limited to the examples illustrated in the figures.

### DETAILED DESCRIPTION

### Introduction and Definitions

This disclosure is written to describe the invention to a person having ordinary skill in the art, who will understand that this disclosure is not limited to the specific examples or embodiments described. The examples and embodiments are single instances of the invention which will make a much larger scope apparent to the person having ordinary skill in the art. Unless defined otherwise, all technical and scientific terms used herein have the same meaning as commonly understood by the person having ordinary skill in the art. It is also to be understood that the terminology used herein is for the purpose of describing examples and embodiments only, and is not intended to be limiting, since the scope of the present disclosure will be limited only by the appended claims.

All the features disclosed in this specification (including any accompanying claims, abstract, and drawings) may be replaced by alternative features serving the same, equivalent, or similar purpose, unless expressly stated otherwise. Thus, unless expressly stated otherwise, each feature disclosed is one example only of a generic series of equivalent or similar features. The examples and embodiments described herein are for illustrative purposes only and that various modifications or changes in light thereof will be suggested to the person having ordinary skill in the art and are to be included within the spirit and purview of this application. Many variations and modifications may be made to the embodiments of the disclosure without departing substantially from the spirit and principles of the disclosure. All such modifications and variations are intended to be included herein within the scope of this disclosure. For example, unless otherwise indicated, the present disclosure is not limited to particular materials, reagents, reaction materials, manufacturing processes, or the like, as such can vary. It is also to be understood that the terminology used herein is for purposes of describing particular embodiments only and is not intended to be limiting. It is also possible in the present disclosure that steps can be executed in different sequence where this is logically possible.

All numeric values are herein assumed to be modified by the term "about," whether or not explicitly indicated. The term "about" generally refers to a range of numbers that one of skill in the art would consider equivalent to the recited value (for example, having the same function or result). In many instances, the term "about" may include numbers that are rounded to the nearest significant figure.

In everyday usage, indefinite articles (like "a" or "an") precede countable nouns and noncountable nouns almost never take indefinite articles. It must be noted, therefore, that, as used in this specification and in the claims that follow, the singular forms "a," "an," and "the" include plural referents unless the context clearly dictates otherwise. Thus, for example, reference to "a support" includes a plurality of supports. Particularly when a single countable noun is listed as an element in a claim, this specification will generally use a phrase such as "a single." For example, "a single support."

Where a range of values is provided, it is understood that each intervening value, to the tenth of the unit of the lower limit (unless the context clearly dictates otherwise), between the upper and lower limit of that range, and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure.

In this specification and in the claims that follow, reference will be made to a number of terms that shall be defined to have the following meanings unless a contrary intention is apparent.

"Absorbent article" refers to devices that absorb and contain liquid, and more specifically, refers to devices that are placed against or in proximity to the body of the wearer to absorb and to contain various exudates discharged from the body. In an example, the absorbent article includes but is not limited to a pant, a taped diaper or a sanitary napkin.

"Fibrous material" refers to a nonwoven material of thin polypropylene (PP) and polyethylene (PE) fibers laid down randomly and point bonded to create web structure and rigidity.

"Roll" refers to multiple layers of fibrous material that are concentrically wound around an inner core.

"Cylinder" refers to multiple rolls that are stacked end to end in an axial direction and wrapped in polymeric covering.

"Axial direction" refers to a direction parallel to a central axis of the cylinder that passes through the inner core of each roll in the cylinder;

"Circumferential direction" refers to a direction around a circumference of each roll of the cylinder.

"Zone of weakness" refers to a region aligned in the axial direction over the polymeric covering, where a tension in the circumferential direction per unit length along the region is greater than along adjacent regions of the polymeric covering.

"Line of weakness" refers to a zone of weakness that takes a linear form in the axial direction over the polymeric covering.

"Open areas" refers to openings formed in the polymeric covering along the line of weakness.

"Land areas" refers to polymeric covering material between the open areas along the line of weakness.

### System for Automatically Removing Polymeric Covering from Cylinder

A system for manufacturing a disposable absorbent article will now be discussed. In one example, the system may be used for performing one or more steps of a method for manufacturing the disposable absorbent article. In one example, these steps may include removing a polymeric covering from a cylinder of multiple rolls of fibrous material. After this removing step, the fibrous material from the rolls may be automatically fed to an absorbent article manufacturing line where the fibrous material is used to manufacture the disposable absorbent article. In an example, the system herein is configured such that during the removal of the polymeric covering, the fibrous material of the multiple rolls is not damaged so to not interfere with the automatic feeding step. Additionally, in another example, the system herein is configured such that other damage to the fibrous material of the rolls is avoided or minimized, including puncturing holes in the fibrous material of the rolls to the extent that it inhibits the ability of the fibrous material to serve various functions in the absorbent article (e.g. as a liquid barrier).

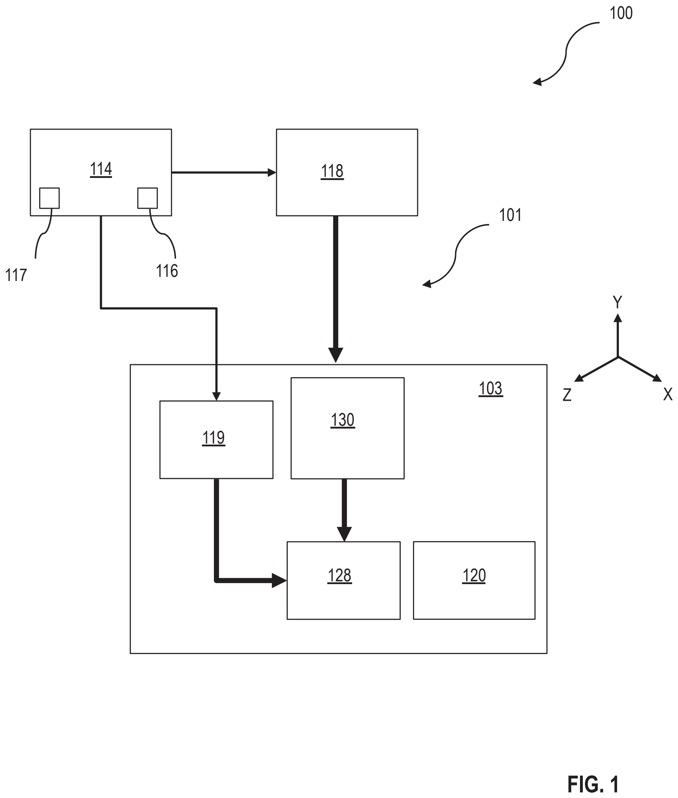

As shown in FIG. 1, the system 100 may include a robot 101 that includes a robot arm 103 which is mechanically coupled with a motor 118. It should be noted that the 1.5 width lines in FIG. 1 indicate communication channels between components of the system 100 whereas 3.5 width lines in FIG. 1 indicate mechanical or thermal channels between components of the system 100. The motor 118 may be configured to move the robot arm 103 in one of multiple directions, such as along one or more of the axes x, y, z shown in FIG. 1. Although one motor 118 is depicted in FIG. 1, in other examples the system may include more than one motor 118 that are configured to respectively move the robot arm 103 along each of the axes x, y z shown in FIG. 1 and/or respectively rotate the robot arm 103 about each of the axes x, y, z shown in FIG. 1. Thus, in one example the system 100 may include up to six motors 118, three of which are configured to move the robot arm 103 along each of the axes x, y ,z and another three of which are configured to rotate the robot arm 103 about each of the axes x, y, z. For those examples, where the system 100 features more than one robot arm 103, the system 100 may include up to six motors 118 that is configured to move each respective robot arm 103.

The system 100 may also feature a controller 114 that may be communicatively coupled with the motor 118. In one example, the controller 114 is configured to transmit signals to the motor 118 in order to cause the motor 118 to move the robot arm 103 in one of a plurality of directions (e.g. along each of the axes x, y z and/or about each of the axes x, y z). In some examples, sensors (not shown) may be provided in the system 100 which measure a position of the robot arm 103 and transmit position data of the robot arm 103 to the controller 114. Based on this received position data, the controller 114 may then transmit a signal to the motor 118 to move the robot arm 103 until the robot arm 103 is in a desired position.

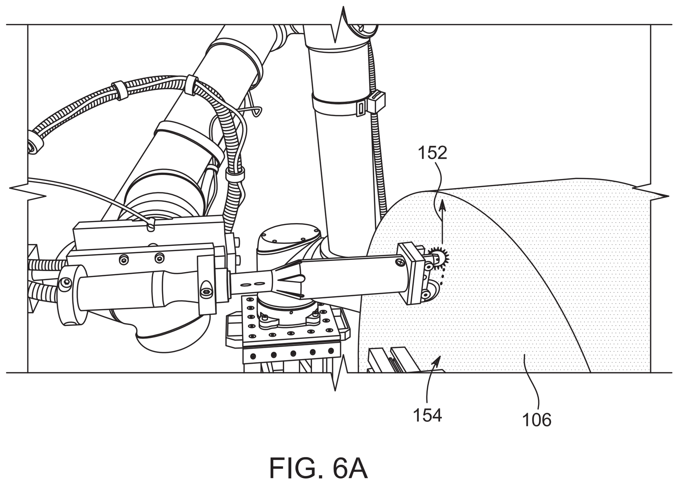

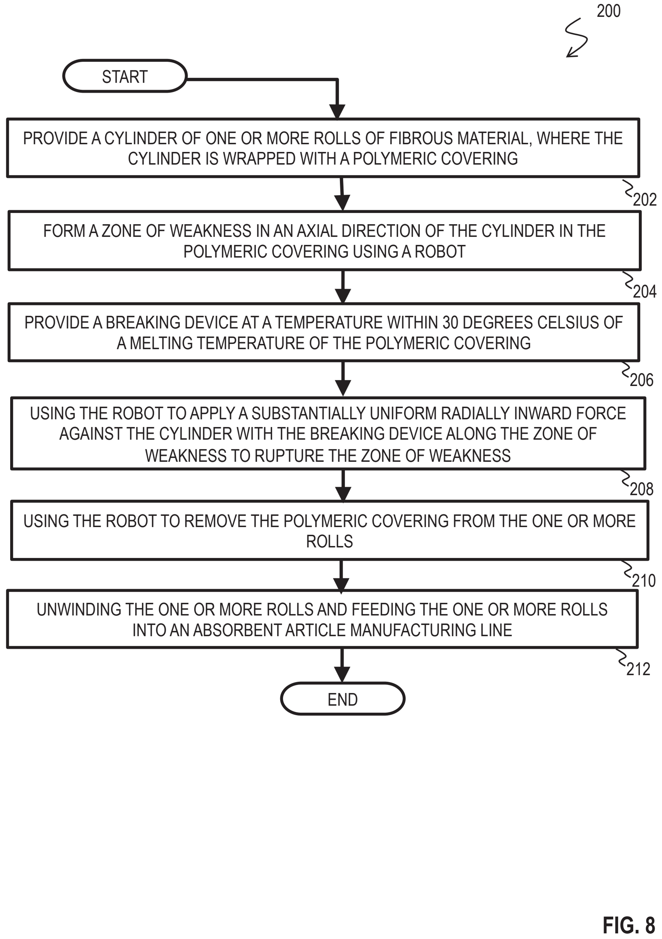

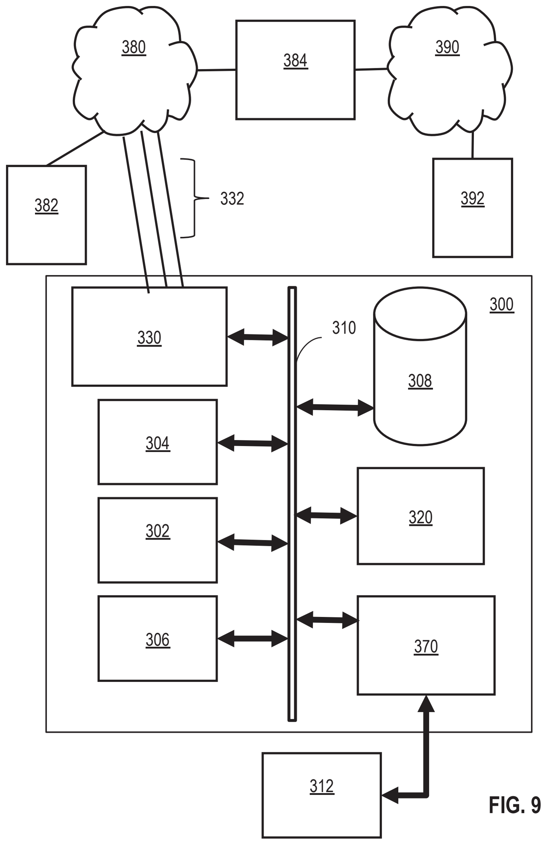

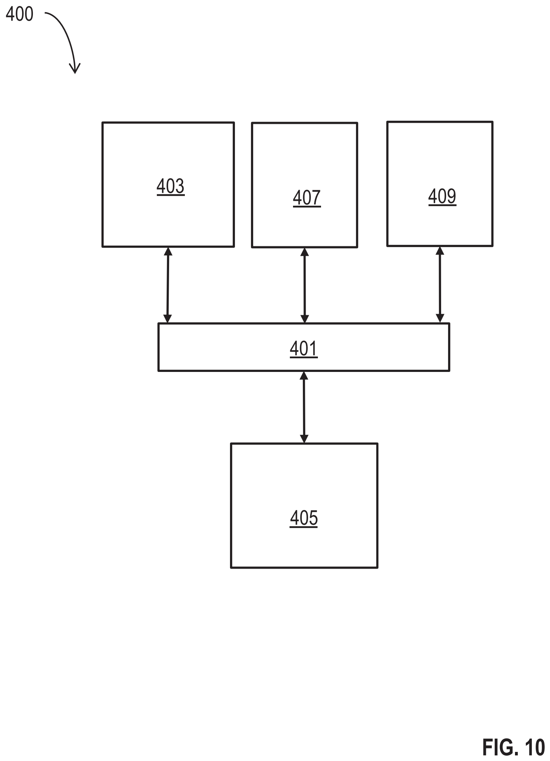

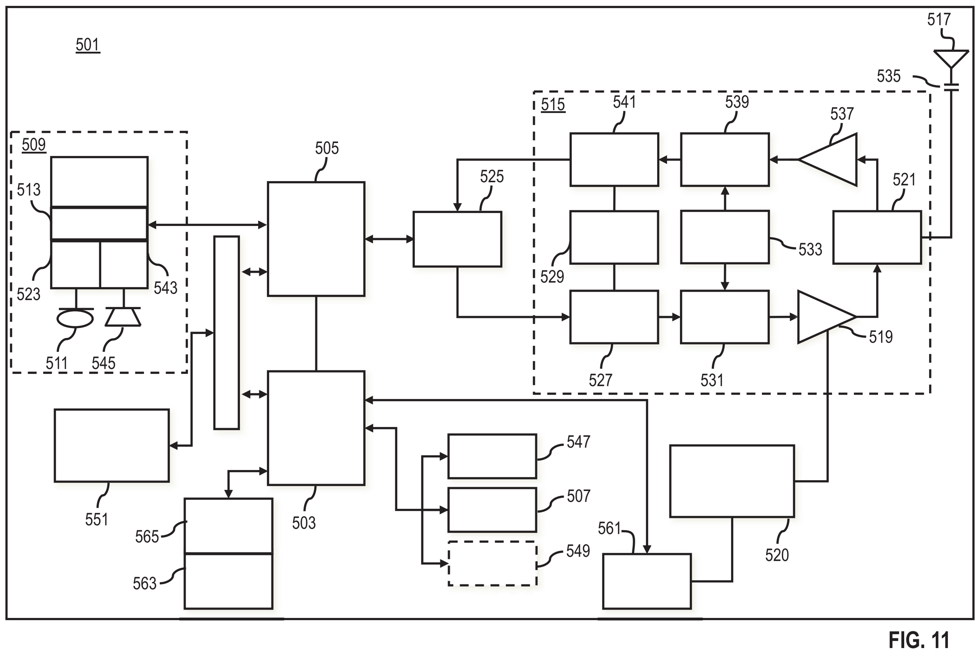

The controller 114 may include a memory 116 that is configured to store data that is used during the operation of the system 100 (e.g. a desired temperature of the breaking device, a desired position of the robot arm 103, etc.). The controller 114 may also include a covering removal module 117 that may include a set of one or more instructions that cause the controller 114 to perform one or more steps of a method, such as the method discussed with respect to FIGS. 6A through 6E herein and/or the method 200 of the flowchart depicted in FIG. 8. In some examples, the processor or controller 114 may be a computer system as described below with reference to FIG. 9, a chip set described below with reference to FIG. 10 or a mobile terminal described below with reference to FIG. 11.

The components of the robot 101 that may be moved by the motor 118 will now be discussed. As shown in FIG. 1, the robot arm 103 may include a perforating device 120 and a breaking device 128. In one example, the system 100 may be configured to move the robot arm 103 along the polymeric covering that packages multiple rolls of fibrous material in a cylinder. As the robot arm 103 moves along the polymeric covering in a direction, the perforating device 120 may be configured to perforate the polymeric covering and form a zone of weakness along the movement direction in the polymeric covering. The breaking device 128 may then be configured to separate or rupture the polymeric covering along the zone of weakness created by the perforating device 120. Consequently, after rupturing or separating the polymeric covering, the polymeric covering can be removed from the cylinder.

Some characteristics of the breaking device 128 will now be discussed. In one example, in order to effectively rupture or separate the polymeric covering along the zone of weakness, a temperature of the breaking device 128 may be controlled. Thus, in this example the system 100 may feature a heating device 119 that is configured to heat the breaking device 128. The heating device 119 may be communicatively coupled with the controller 114 and configured to adjust the temperature of the breaking device 128 based on one or more signals received from the controller 114. Although not depicted in FIG. 1, in one example the system 100 may include temperature sensors on one or both of the heating device 119 and/or breaking device 128 which may provide feedback to the controller 114 of the temperature of the breaking device 128. Based on this feedback to the controller 114, the controller 114 may adjust the signals transmitted to the heating device 119 until the temperature of the breaking device 128 is at a desired temperature. In one example, the desired temperature of the breaking device 128 is a temperature within a certain range (e.g. 150 degrees Celsius) of a melting temperature of the polymeric covering (e.g. about 95 degrees Celsius). In one example, the desired temperature of the breaking device 128 is about 200 degrees Celsius (or within a range from about 180 degrees Celsius to about 220 degrees Celsius) and thus in this example, the desired temperature of the breaking device 128 is within about 100 degrees Celsius of the melting temperature of the polymeric covering. In yet another example, the desired temperature of the breaking device 128 may be equal to or greater than the melting temperature of the polymeric covering (e.g. about 95 degrees Celsius).

Another characteristic of the breaking device 128 may include a force control feature, such that when the breaking device 128 is moving across an uneven surface (e.g. variations in an outer diameter of the cylinder along the zone of weakness) the breaking device 128 does not impart more than a maximum threshold force on the surface. In one example, the system 100 may include a force control device 130 that is provided which may bias the breaking device 128 towards a surface (e.g. zone of weakness in the polymeric covering) and controls a force imparted by the breaking device 128 on the surface. In one example, the force control device 130 may be a spring. In another example, the force control device 130 may be load cells in robotic joints to control loading. In yet another example, the force control device 130 may be a pneumatic air cylinder configured to deliver constant force. In one example, the force control device 130 is configured to impart a force on the breaking device 128 based on a displacement of the breaking device 128 due to variations in the uneven surface that the breaking device 128 is moving across.

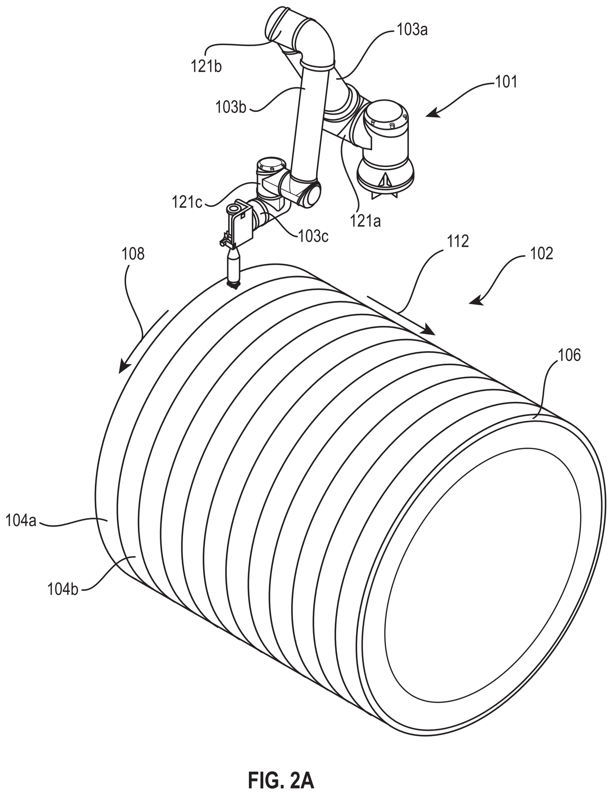

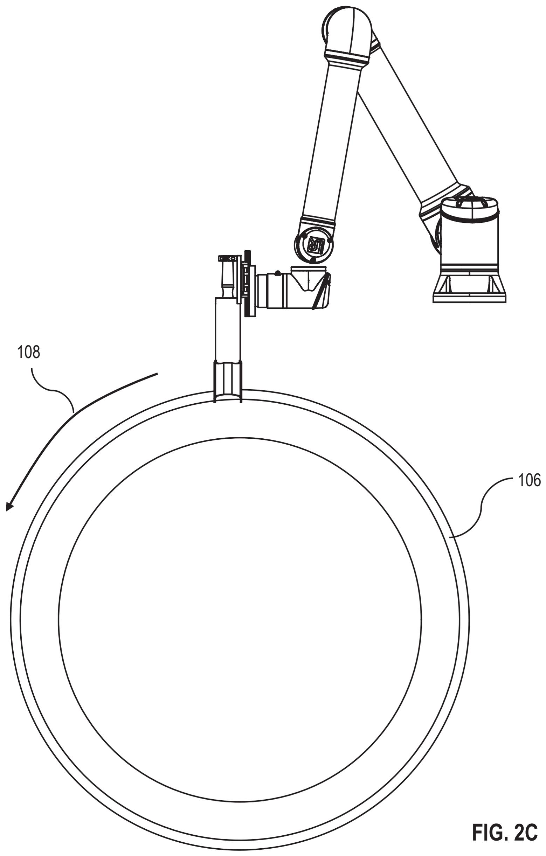

The use of the system 100 in conjunction with a cylinder of multiple rolls of fibrous material wrapped in a polymeric covering will now be discussed. FIGS. 2A through 2C are various views of an example of the system 100 of FIG. 1 being used to remove a polymeric covering 106 from a cylinder 102 including a plurality of rolls 104a, 104b of fibrous material. For ease of illustration, only two of the rolls 104a, 104b within the cylinder 102 are labeled. In some examples, the cylinder 102 may include more than three rolls 104 of fibrous material packaged in the polymeric covering 106. The cylinder 102 of the multiple rolls 104a, 104b may be at least partially wrapped with the polymeric covering 106. As shown in FIG. 2A, the polymeric covering 106 may be wrapped around the rolls 104a, 104b so to have a tension in the circumferential direction 108 of the cylinder 102. In some examples, the polymeric covering 106 may have more than one layer. In yet another example, the polymeric covering 106 may have a different number of layers along a top of the cylinder 102 as compared to along opposite sides of the cylinder 102. In still another example, the polymeric covering 106 may have a different number of layers over each of the multiple rolls 104a, 104b within the cylinder 102.

In one example, each of the multiple rolls 104a, 104b may feature the fibrous material that may be a nonwoven material. In yet another example, the fibrous material may include polyfilms or laminates. In yet another example, the fibrous material of the multiple rolls 104a, 104b may have a melting temperature (e.g. about 140 degrees Celsius) that is above the melting temperature (e.g. about 95 degrees Celsius) of the polymeric covering 106 (e.g. by at least 10 degrees Celsius).

As shown in FIG. 2A, the system 100 may include the robot 101 that may have multiple robot arms 103a, 103b, 103c which are each separated from one another with a respective joint 121a, 121b, 121c. In an example, each of the joints 121a, 121b, 121c may feature one or more motors 118 that are configured to rotate an adjacent robot arm of that joint about one or more of the x, y, z axes in FIG. 1.

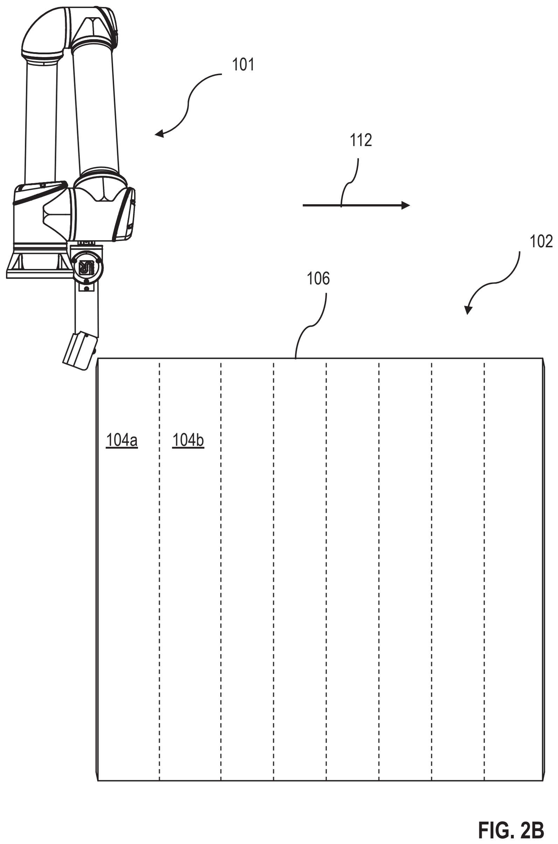

As shown in FIGS. 2A and 2B, in one example the robot 101 may move the robot arm 103c to which the perforating device 120 and breaking device 128 are coupled in an axial direction 112 along a top of the cylinder 102. As the perforating device 120 is moved in the axial direction 112 along the top of the cylinder 102, the perforating device 120 may create the zone of weakness by perforating holes in the polymeric covering 106 along the axial direction 112. In this example, as the breaking device 128 is moved in the axial direction 112 along with the perforating device 120, the breaking device 128 (e.g. that may be heated to a desired temperature) may rupture or separate the polymeric covering 106 along the zone of weakness formed by the perforating device 120. This may result in separation of the polymeric covering 106 from the multiple rolls 104a, 104b of the cylinder 102 along the top of the cylinder 102. In another example, in order to fully separate the polymeric covering 106 from the multiple rolls 104a, 104b of the cylinder 102, the perforating device 120 and breaking device 128 may also be moved along the opposite sides of the cylinder 102 so to similarly separate the polymeric covering 106 from the rolls of the cylinder along the opposite sides of the cylinder 102.

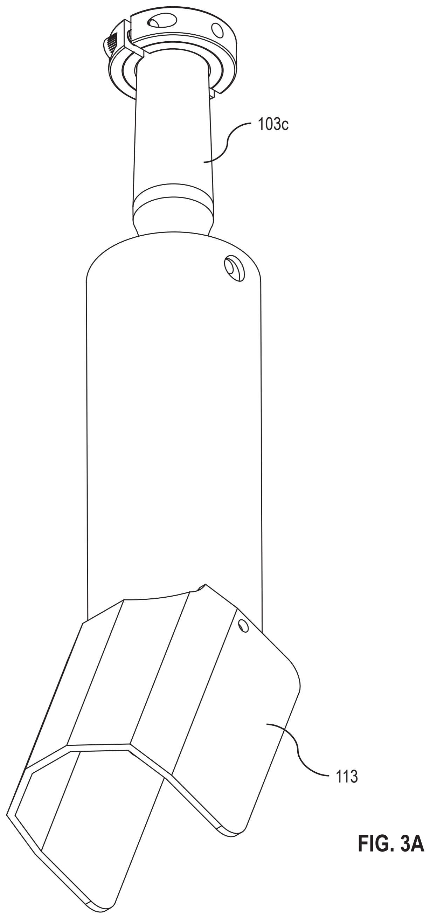

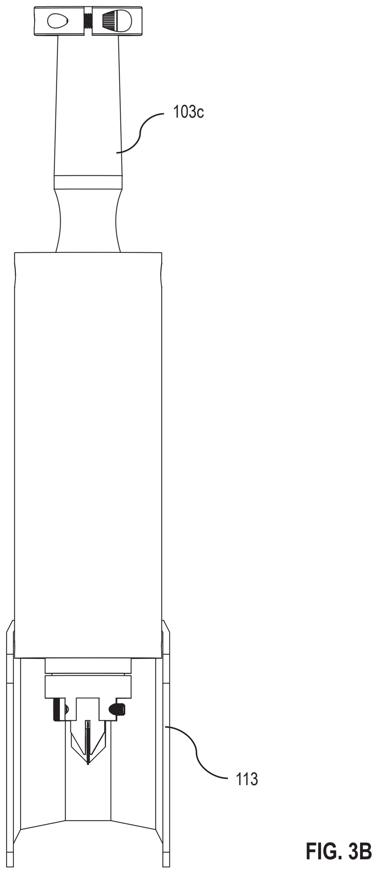

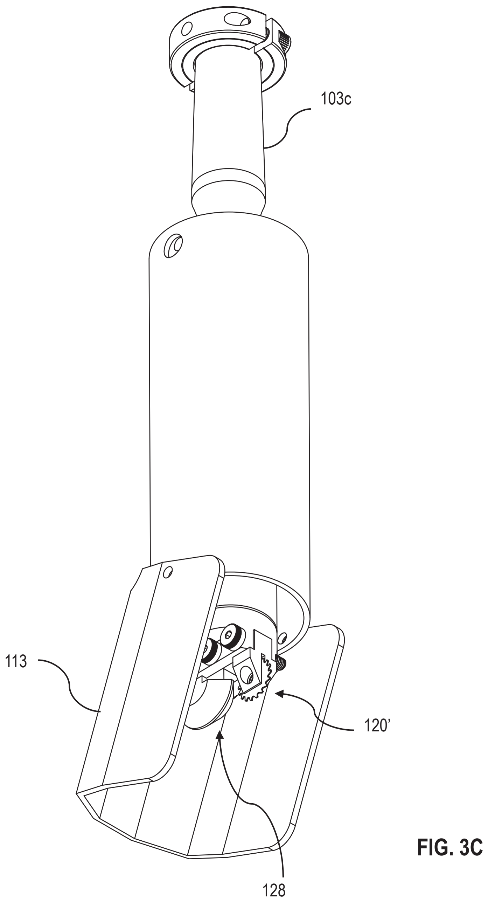

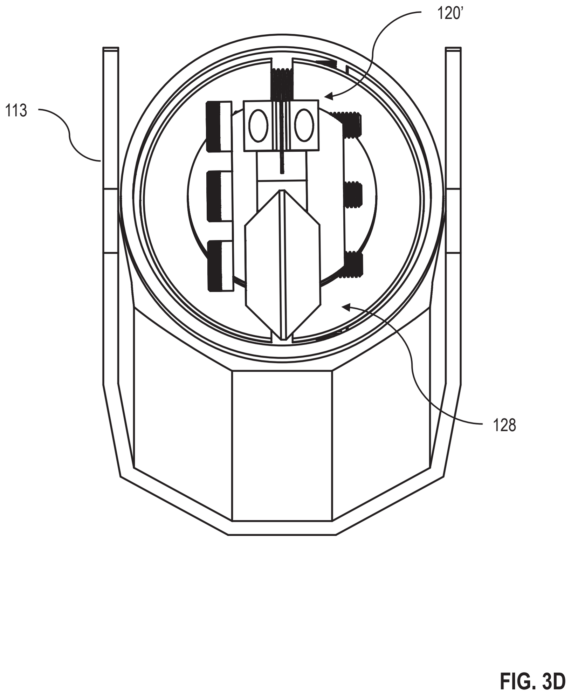

Some features of the robot arm to which the perforating device 120 and breaking device 128 are coupled is now discussed. FIGS. 3A through 3D are various views of an example of an end of a robot arm 103c of the system 100 of FIG. 2A. In one example, the perforating device 120 may be a perforating wheel 120' (FIG. 3C). In this example, the perforating wheel 120' and the breaking device 128 may be mounted to an end of the robot arm 103c as shown in FIG. 3C. A cover 113 may be provided at the end of the robot arm 103c which may advantageously protect the perforating wheel 120' and breaking device 128 when the system 100 is not in use. In one example, the cover 113 may be pivotably mounted to the end of the robot arm 103c such that it can be rotated from a first position (FIG. 3B) where the perforating wheel 120' and breaking device 128 are protected when the system 100 is not in use to a second position (not shown) where the perforating wheel 120' and breaking device 128 may be exposed and are thus positioned to form the zone of weakness and separate the polymeric covering 106 along the zone of weakness when moved in the axial direction 112 along the cylinder 102.

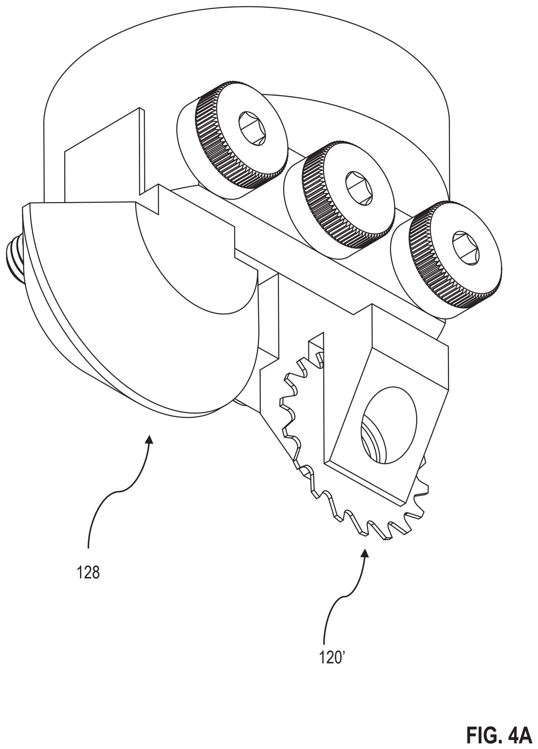

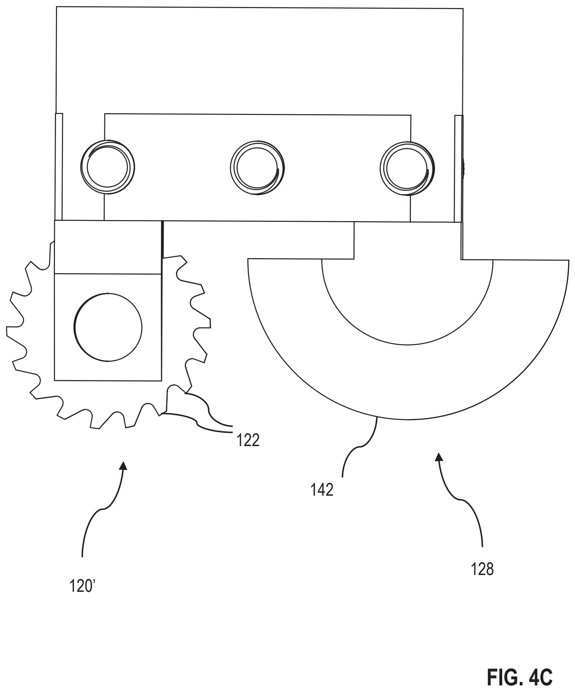

Some features of specific examples of the perforating device 120 and breaking device 128 will now be discussed. FIGS. 4A through 4D are various views of an example of a breaking device 128 and a perforating device 120 at the end of the robot arm 103c of FIGS. 3A through 3D. As previously discussed, in one example the perforating device 120 may be a perforating wheel 120'. As depicted in FIG. 4C, the perforating wheel 120' may be rotatable about an axis of rotation and feature one or more spokes 122. In this example, as the robot arm 103c is moved in the axial direction 112 along the polymeric covering 106, the spokes 122 rotate and perforate the polymeric covering 106 to create a zone of weakness that may be a line of weakness along the axial direction 112. In an example, the line of weakness may feature perforations or open areas based on puncturing of the polymeric covering 106 with the spokes 122 and land areas positioned between consecutive open areas. In an example, circumferential spacing of the spokes 122 is adjusted to correspond to a desired spacing of perforations or open areas along the axial direction 112. In an example, the circumferential spacing of the spokes 122 may be from about 10 degrees to about 30 degrees so to correspond to a desired spacing of the open areas of about 3 mm to about 5 mm. Similarly, in an example, a width of the spokes 122 (e.g. adjacent a base thereof) may be selected so to correspond to a desired width of the open areas in the polymeric covering 106 (e.g. measured along the axial direction 112). In an example, the spoke width may be from about 1/2 mm to about 1 1/2 mm to correspond to the desired width of the open areas from about 1 mm to about 2 mm. In one example, the perforating wheel 120' may be provided by various manufacturers, such as Steward of America^{®} (Simpsonville, SC).

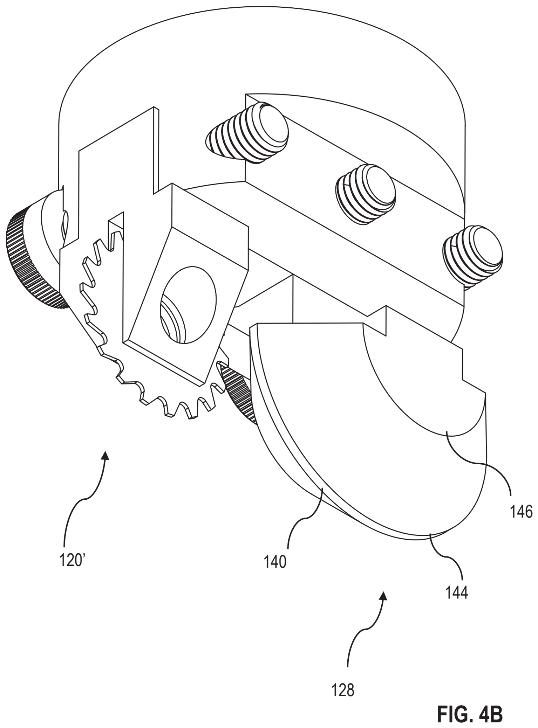

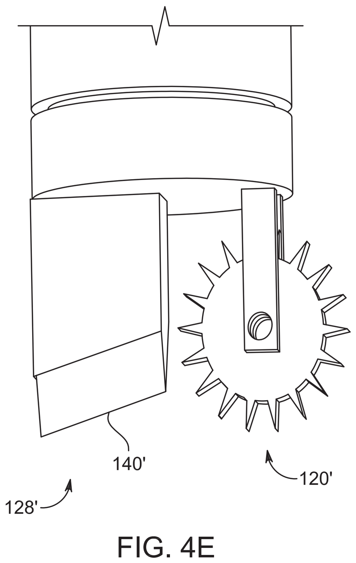

As further depicted in FIG. 4B, the breaking device 128 may feature a first end 144 with a pointed projection 140 and a second end 146 spaced a distance apart from the first end 144. In an example, the breaking device 128 may be a wheel, where the pointed projection 140 at the first end 144 may be along an outer circumference 142 (FIG. 4C) of the wheel. Additionally, as shown in FIG. 4B, the second end 146 may be positioned radially inward of the outer circumference 142 of the wheel. In yet another example, a thickness of the breaking device 128 may be less at the first end 144 with the pointed projection 140, as compared with the second end 146. Thus, in this example, a thickness of the breaking device 128 may be tapered from the second end 146 to the first end 144. However, in another example, the breaking device 128 need not be in the form of a wheel. FIG. 4E is a side view of an example of a breaking device 128' and a perforating wheel 120' at the end of the robot arm 103c of FIGS. 3A through 3D. In the example of FIG. 4E, unlike the breaking device 128 wheel of FIGS. 4A through 4D, the breaking device 128' includes a blade having a tapered width from a first end at the pointed projection 140' to a second end spaced apart from the pointed projection 140'.

In one example, a force control device 130 may be provided for the breaking device 128 (e.g. wheel with the pointed projection 140) and for the perforation wheel 120'. In this example, the breaking device 128 (e.g. wheel with the pointed projection 140) may feature a force control device 130 (e.g. load cells in robotic joints) whereas the perforation wheel 120' may feature a separate or independent force control means 130 (e.g. spring) than the force control device 130 of the breaking device 128. Accordingly, in this example, the perforation wheel 120' may be on a spring loaded system so it can float relative to the breaking device 128 as it travels over roll transitions that may create an uneven surface. In this example, the load on the breaking device 128 may be separately controlled by the robot joints as the breaking device 128 (e.g. wheel with the pointed projection 140) passes over roll transitions that create an uneven surface.

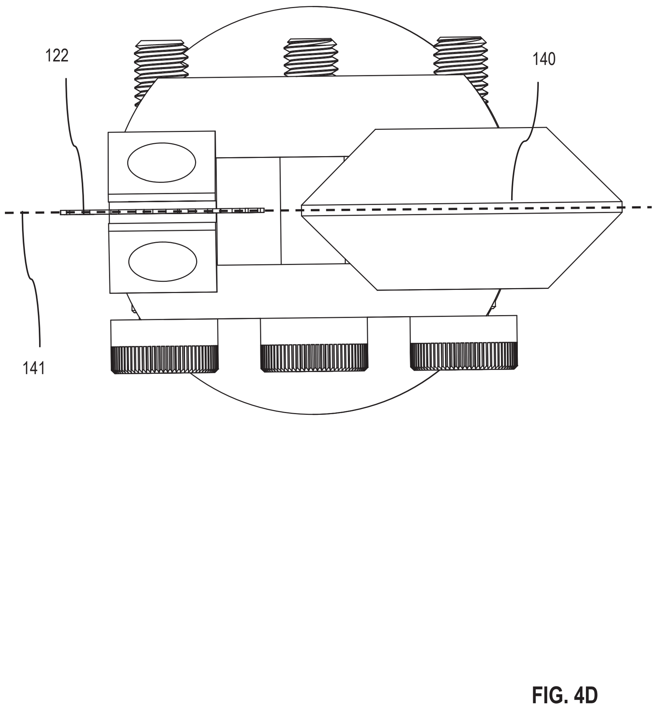

As shown in FIG. 4D, in one example the spokes 122 of the perforation wheel 120' and the pointed projection 140 of the breaking device 128 are aligned in a common plane 141. This may advantageously ensure that the pointed projection 140 is configured to rupture or separate the polymeric covering 106 along the line of weakness in the polymeric covering 106 formed by the spokes 122 of the perforation wheel 120'.

### Method for Automatically Removing Polymeric Covering From Cylinder

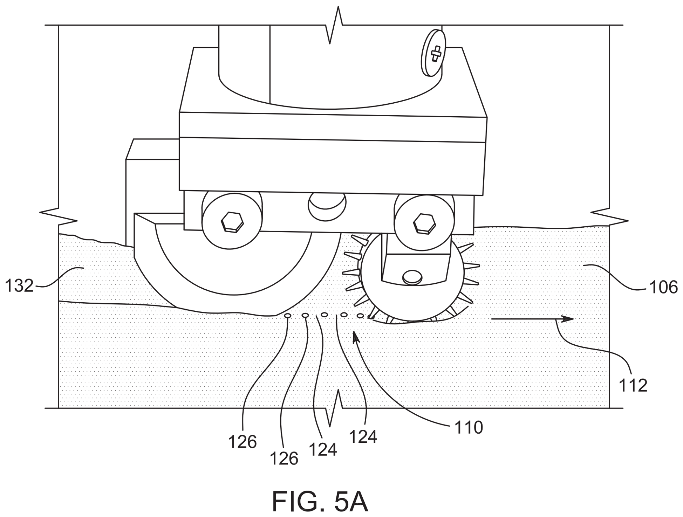

A method for using the system of FIG. 1 to automatically remove the polymeric covering 106 from the cylinder 102 is now discussed. FIG. 5A is a side view of an example of the perforating device 120 forming a line of weakness 110 in the polymeric covering 106 and a breaking device 128 rupturing the polymeric covering 106 along the line of weakness 110. In an example, as shown in FIG. 5A, the line of weakness 110 may include a plurality of open areas 126 and a respective plurality of land areas 124 therebetween along the axial direction 112. The open areas 126 may be formed by the spokes 122 of the perforating wheel 120' puncturing the polymeric covering 106 along the axial direction 112. As the perforating wheel 120' moves in the axial direction 112 over the covering 106, the perforating wheel 120' rotates and thus the spokes 122 form the open areas 126 along the axial direction 112.

One advantage of the formed zone of weakness (e.g. line of weakness 110) in the polymeric covering 106 by the perforating device 120 is now discussed. As depicted in FIG. 2A, in an example the polymeric covering 106 is wrapped around the multiple rolls 104a, 104b of the cylinder 102 with a certain tension in the circumferential direction 108. It was recognized herein that the effort required to rupture or separate the polymeric covering 106 along the axial direction 112 (e.g. with the breaking device 128) depends on the circumferential tension of the polymeric covering 106 along the axial direction 112. For example, the greater the circumferential tension of the polymeric covering 106 along the axial direction 112, the lesser is the effort required to rupture or separate the polymeric covering 106 along the axial direction 112. Thus, it was recognized that it may be advantageous to form the zone of weakness (e.g. line of weakness 110) along the axial direction 112, since this would result in the same circumferential tension being applied across a reduced length of polymeric covering 106 (e.g. the land areas 124 along the line of weakness 110) in the axial direction 112. Consequently, this may increase the circumferential tension per unit length along the axial direction 112 and consequently reduce the required effort to rupture or separate the polymeric covering 106 along the axial direction 112 with the breaking device 128. In another example, other means may be employed to increase the circumferential tension along the axial direction 112. For example, one or more vacuum components may be used to suction the polymeric covering 106 on opposite sides of the axial direction 112 so to increase the circumferential tension in a direction along the axial direction 112 and thus reduce the required effort to rupture the polymeric covering 106 along the axial direction 112 (e.g. with the breaking device 128). This example may be employed in scenarios where the circumferential tension along the axial direction 112 is insufficient to reduce the necessary force to be imparted on the polymeric covering 106 with the breaking device 128 in order to rupture the polymeric covering 106.

Although FIG. 5A depicts the line of weakness 110 formed by the perforating wheel 120', in other examples the perforating device 120 of the system 100 may be employed to form any zone of weakness in the polymeric covering 106 along the axial direction 112, other than a single line of weakness 110. Thus, in some examples, the perforating device 120 may form a zone of weakness that includes multiple lines of weakness 110 that are each parallel to one another. In this example, such multiple lines of weakness 110 that are parallel to each other may be formed by a pair of side-by-side perforation wheels 120' at the end of the robot arm 103c. In still other examples, the perforating device 120 may form a zone of weakness that does not feature one or more lines of weakness 110 but instead features any arrangement of open areas 126 and land areas 124 along the axial direction 112 which increases the circumferential tension per unit length along the axial direction 112.

In an example, the breaking device 128 depicted in FIG. 5A may be heated (e.g. by the heating device 119) to a temperature above the melting temperature of the polymeric covering 106 (e.g. about 95 degrees Celsius) and/or within a certain range (e.g. about 150 degrees Celsius) of the melting temperature of the polymeric covering 106.

In an example, using the robot 101, the breaking device 128 may apply a substantially uniform radially inward force against the cylinder 102 and the polymeric covering 106 along the line of weakness 110 to rupture or separate the polymeric covering 106 along the line of weakness 110, resulting in the ruptured covering 132 shown in FIG. 5A. In one example, the rupturing of the zone of weakness by the breaking device 128 may occur at a certain rate, such as between about 3 inches per second to about 8 inches per second. In one example, the breaking device 128 is a severing device. In yet another example, the breaking device 128 is a melting device (e.g. heated by the heating device 119 of FIG. 1). In one example, the substantially uniform radially inward force may be applied by the breaking device 128 in a direction that is parallel with and/or aligned with the one or more lines of weakness 110 formed in the axial direction 112. In one example, where a single line of weakness 110 is formed in the polymeric covering 106, the breaking device 128 may apply the substantially uniform radially inward force along a direction that is aligned with the single line of weakness 110. In yet another example, where a plurality of lines of weakness 110 are formed in the polymeric covering 106, the breaking device 128 may be apply the substantially uniform radially inward force along a direction that is parallel with and/or between the plurality of lines of weakness 110.

As previously discussed, in one example the step of forming the line of weakness 110 may advantageously increase the circumferential tension in the polymeric covering 106 per unit length along the axial direction 112. It was recognized that this may advantageously reduce the required effort to rupture or separate the polymeric covering 106 (e.g. with the breaking device 128) along the axial direction 112. Consequently, this increase in the circumferential tension per unit length along the axial direction 112 may advantageously reduce the extent of the substantially uniform radially inward force to be applied by the breaking device 128, in order to rupture or separate the polymeric covering 106 along the line of weakness 110.

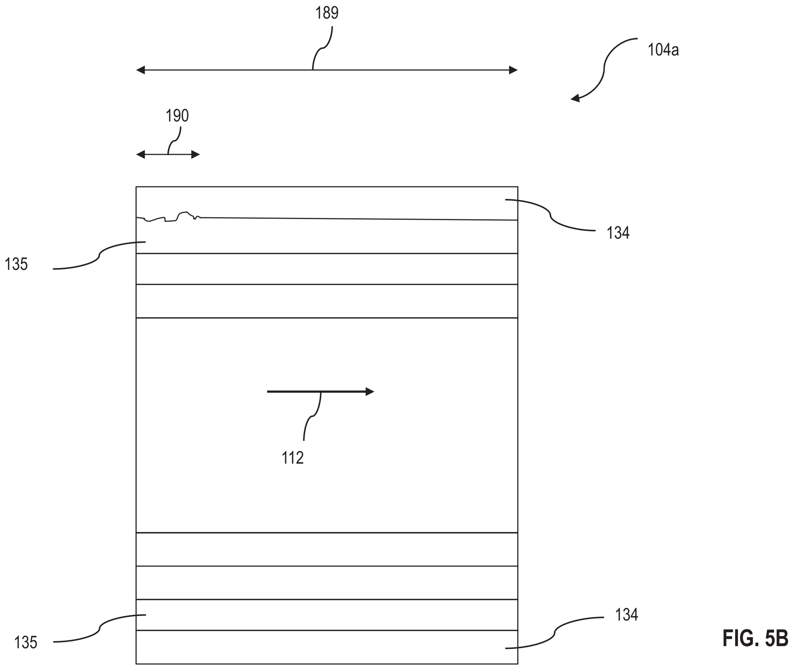

As previously discussed, in one example the system 100 may feature the force control device 130 (e.g. spring) that ensures that the substantially uniform radially inward force applied by the breaking device 128 against the cylinder 102 does not exceed a maximum threshold force. Thus, in this example, the force control device 130 ensures that the breaking device 128 does not impart a force above the maximum threshold force due to variations in an outer diameter of the cylinder 102. In some examples, these variations in the outer diameter of the cylinder 102 may be due to a variation in the number of layers in the polymeric covering 106 along the axial direction 112. In other examples, these variations in the outer diameter of the cylinder 102 may be due to a different number of fibrous material layers in consecutive rolls 104a, 104b of the cylinder 102 and/or different concentric alignment of consecutive rolls 104a, 104b within the cylinder 102. In these examples, the force control device 130 is configured to vary a biasing force applied by the breaking device 128 on the cylinder 102 based on a displacement of the breaking device 128 due to the variations in the outer diameter of the cylinder 102. In one example, the force control device 130 is a spring having a spring constant whose value is selected (e.g. about 1.5 N/m) so that the maximum threshold force does not exceed a certain value (e.g. about 25 N). In other examples the force control device 130 may feature one or more sensors or other components that are communicatively coupled with the controller 114 which processes the data from these components and transmits one or more signals to the force control device 130 to selectively vary the biasing force applied by the breaking device 128 on the cylinder 102 due to these variations in the outer diameter of the cylinder 102. For example, the sensors may detect a displacement of the breaking device 128 away from the cylinder 102, due to the breaking device 128 impacting the uneven outer diameter of the cylinder 102 and in response to receiving this data the controller 114 may transmit a signal to the force control device 130 to reduce the imparted force by the breaking device 128 on the cylinder 102. As discussed with respect to FIG. 5B below, it was recognized that the force control device 130 herein may provide certain advantages, including minimizing risk of damage to the outer layers of the rolls and thus minimizing impact on subsequent automated steps of the disposable absorbent article manufacturing method.

The method disclosed herein minimizes a risk of damage to the fibrous material of the rolls 104a, 104b in the cylinder 102. FIG. 5B is cross-sectional view of an example of a roll 104a in the cylinder 102 showing multiple outer layers 134, 135 of the roll 104a fused together over a fused width portion 190 of the roll width 189. As previously discussed, as the breaking device 128 moves in the axial direction 112 and ruptures or separates the polymeric covering 106, it may make contact with the outer layers 134, 135 of the roll 104a. Thus, in this example, the breaking device 128 may transfer heat to the outer layers 134, 135 of the roll 104a. If the breaking device 128 overheats or transfers an excess amount of heat to the outer layers 134, 135 of the roll 104a, they may fuse or melt together or may get cut, severed or damaged over the fused width 190 portion of the roll width 189, as shown in FIG. 5B. As previously discussed, if the ratio between the fused width 190 and the roll width 189 is greater than a threshold amount (e.g. 20 %) then this fusion between the outer layers 134, 135 of the roll 104a may hinder subsequent steps of the disposable absorbent article manufacturing method, such as an automatic pick up step where the outer layer 134 is automatically grabbed by a robot and fed into the absorbent article manufacturing line. If the fused width 190 is too large, then the automatic pick up step cannot be performed, since the two outer layers 134, 135 are fused together to the extent that the robot cannot automatically pickup the outer layer 134. The method disclosed herein may be designed so to ensure that the ratio of the fused width 190 to the roll width 189 is less than the threshold amount (e.g. about 20% or in a range from about 10% to about 30%). Although two outer layers 134, 135 are depicted in FIG. 5B, the fused width 190 could be measured between any two adjacent layers of the roll 104a. Additionally, although FIG. 5B depicts that the roll 104a has four layers, this is merely for purposes of illustration and in most contexts the roll 104a has far more than four layers of fibrous material.

In some examples, one or more parameters of the method may be selected so that a thermal transfer rate from the breaking device 128 to the outer layers 134, 135 of the roll 104a does not exceed a maximum threshold rate. In one example, these parameters include but are not limited to one or more of the temperature of the breaking device 128 (e.g. based on the heating device 119) and/or a speed of the breaking device 128 being moved across the cylinder 102 in the axial direction 112 (e.g. based on the motor 118). In an example, these parameter values may be stored in the memory 116 of the controller 114 and retrieved by the controller 114 during the method steps herein. In an example, the maximum threshold rate of the thermal transfer rate is about 300 Watts. In one example, this selection of the parameters so to ensure that the maximum threshold rate is not exceeded may advantageously result in the fused width 190 being less than the desired ratio (e.g. 20%) of the roll width 189.

The method will now be discussed herein, where a sequential order of steps or stages of the method are discussed. FIGS. 6A through 6E are perspective views of an example of various stages of a method for automatically removing the polymeric covering 106 from the cylinder 102 using the system 100 of FIG. 1. In this example, the polymeric covering 106 surrounds not only the rolls 104 along a top of the cylinder 102 but also along adjacent first and second sides of the cylinder 102 (e.g. to a certain extent that is less than the radius of the cylinder 102).

As shown in FIG. 6A, in a first stage of the method, the robot 101 may automatically move the perforating device 120 and breaking device 128 in a first path along a first radial direction 152 along a first side 154 of the cylinder 102 which is covered by the polymeric covering 106. As shown in FIG. 6A, in this example the controller 114 may be configured to transmit signals to the one or more motors 118 to cause the perforating device 120 and breaking device 128 to move in the first radial direction 152. The perforating device 120 may form the line of weakness 110 along the first side 154 of the cylinder 102 after which the breaking device 128 may rupture the line of weakness 110 along the first side 154 of the cylinder 102. In one example, the first radial direction 152 may be along the first side 154 of the cylinder and may be perpendicular to the axial direction 112 along the top of the cylinder 102.

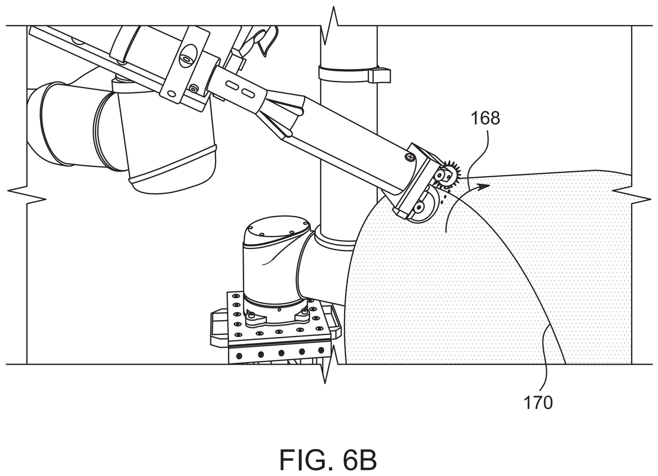

As shown in FIG. 6B, in a second stage of the method, the robot 101 may automatically move the perforating device 120 and breaking device 128 in a second path along a mixed direction 168 over a first corner 170 of the cylinder 102. In one example, the mixed direction 168 is a combination of the first radial direction 152 and the axial direction 112. As shown in FIG. 6B, in this example the controller 114 may be configured to transmit signals to the one or more motors 118 to cause the perforating device 120 and breaking device 128 to move in the mixed direction 168 over the first corner 170 of the cylinder 102. The perforating device 120 may form the line of weakness 110 over the first corner 170 of the cylinder 102 after which the breaking device 128 may rupture the line of weakness 110 over the first corner 170 of the cylinder 102. In one example, the force control device 130 may be configured to ensure that if the breaking device 128 impacts the roll 104a, 104b over the first corner 170, that the breaking device 128 may deflect outward (away from the first corner 170) so to ensure that the breaking device 128 does not damage the layers of the roll 104a at the first corner 170 of the cylinder 102.

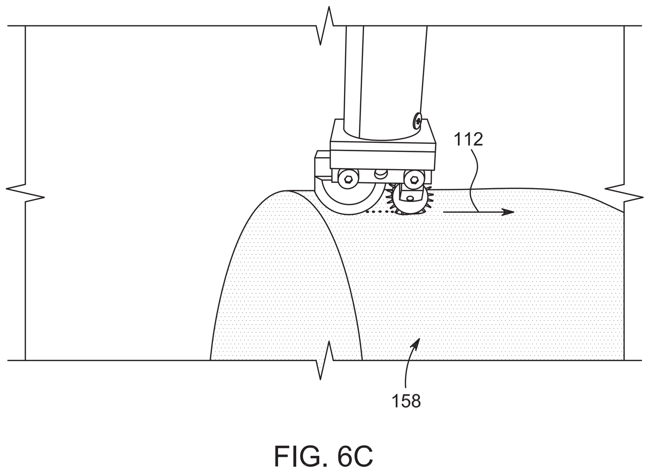

As shown in FIG. 6C, in a third stage of the method, the robot 101 may automatically move the perforating device 120 and breaking device 128 in a third path along the axial direction 112 along a top 158 of the cylinder 102 which is covered by the polymeric covering 106. As shown in FIG. 6C, in this example the controller 114 may be configured to transmit signals to the one or more motors 118 to cause the perforating device 120 and breaking device 128 to move in the axial direction 112. The perforating device 120 may form the line of weakness 110 along the top 158 of the cylinder 102 after which the breaking device 128 may rupture the line of weakness 110 along the top 158 of the cylinder 102.

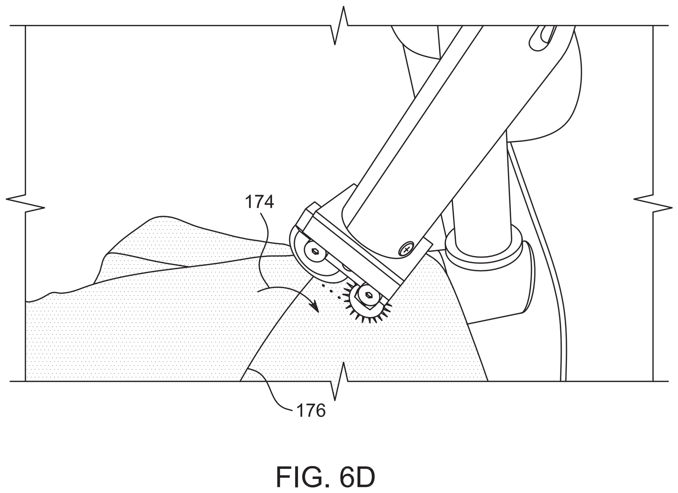

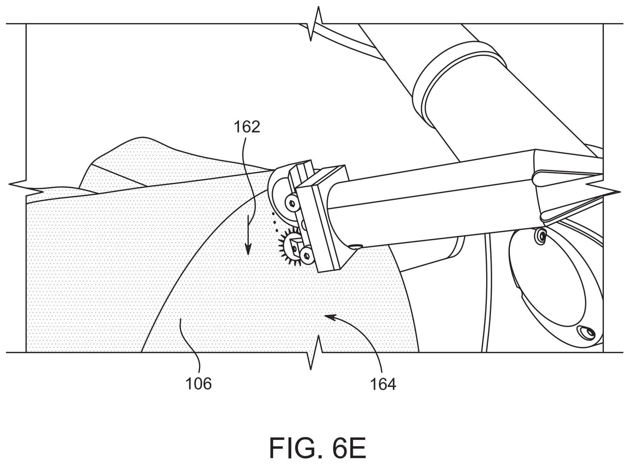

As shown in FIG. 6D, in a fourth stage of the method, the robot 101 may automatically move the perforating device 120 and breaking device 128 in a fourth path along a mixed direction 174 over a second corner 176 of the cylinder 102. In one example, the mixed direction 174 is a combination of the second radial direction 162 (FIG 6E) and the axial direction 112. As shown in FIG. 6D, in this example the controller 114 may be configured to transmit signals to the one or more motors 118 to cause the perforating device 120 and breaking device 128 to move in the mixed direction 174 over the second corner 176 of the cylinder 102. The perforating device 120 may form the line of weakness 110 over the second corner 176 of the cylinder 102 after which the breaking device 128 may rupture the line of weakness 110 over the second corner 176 of the cylinder 102. In one example, the force control device 130 may be configured to ensure that if the breaking device 128 impacts the roll 104a, 104b over the second corner 176, that the breaking device 128 may deflect outward (away from the second corner 176) so to ensure that the breaking device 128 does not damage the layers of the roll at the second corner 176 of the cylinder 102.

As shown in FIG. 6E, in a fifth stage of the method, the robot 101 may automatically move the perforating device 120 and breaking device 128 in a fifth path along a second radial direction 162 along a second side 164 of the cylinder 102 which is covered by the polymeric covering 106. The second radial direction 162 may be opposite in direction to the first radial direction 152. As shown in FIG. 6E, in this example the controller 114 may be configured to transmit signals to the one or more motors 118 to cause the perforating device 120 and breaking device 128 to move in the second radial direction 162 along the second side 164 of the cylinder 102. The perforating device 120 may form the line of weakness 110 along the second side 164 of the cylinder 102 after which the breaking device 128 may rupture the line of weakness 110 along the second side 164 of the cylinder 102. In one example, the second radial direction 162 may be along the second side 164 of the cylinder and may be perpendicular to the axial direction 112 along the top of the cylinder 102.

In some examples, the method may be performed where the robot 101 automatically moves the perforating device 120 and breaking device 128 in the radial direction 152 (FIG. 6A), the axial direction 112 (FIG. 6C) and the radial direction 162 (FIG. 6E) and thus does not move the perforating device 120 and breaking device 128 in the mixed directions 168, 174 (FIGS. 6B, 6D). In these examples, the robot 101 automatically moves the perforating device 120 and breaking device 128 in these directions such that the polymeric covering 106 is ruptured over the first and second corners 170, 176 of the cylinder 102.

In another example, in each of the above discussed stages of the method, each of the paths to which the robot 101 automatically moves the perforating device 120 and breaking device 128 including the radial direction 152, the mixed direction 168, the axial direction 112, the mixed direction 174 and the radial direction 162 may all encompass a common plane. In some examples, the memory 116 of the controller 114 may store data indicating this common plane (e.g. cartesian coordinate values for the x, y, z axes) such that during the automatic moving steps, the controller 114 transmits signals to the motors 118 during these method stages so to ensure that the perforating device 120 and breaking device 128 remain in the common plane as they traverse each of the five paths along the five respective directions.

In yet another example, the system 100 may include an imaging device (e.g. camera) that captures image data of the cylinder 102 as the method stages are performed and the controller 114 may process the image data from the imaging device so to control the position of the perforating device 120 and breaking device 128 in subsequent method stages. For example, during the first stage of moving along the first radial direction 152, the imaging device may capture image data indicating a location of the line of weakness 110 along the first side 154 of the cylinder 102. The controller 114 may then process this image data and use this location data of the line of weakness 110 along the first side 154 of the cylinder 102 so to ensure that the line of weakness 110 formed over the first corner 170 of the cylinder 102 (FIG. 6B) and/or over the top 158 of the cylinder 102 (FIG. 6C) remain aligned with and/or in the same plane as the line of weakness 110 along the first side 154 of the cylinder 102.

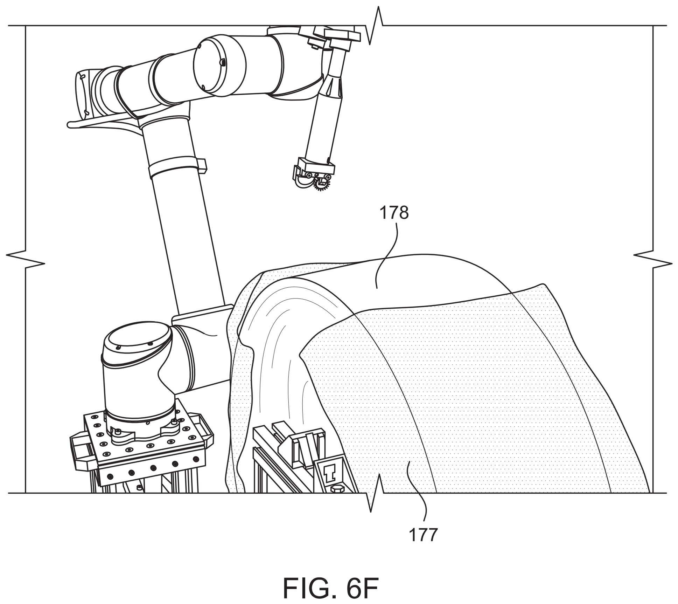

After the various stages of the method are performed, the polymeric covering 106 is separated from the rolls 104 of the cylinder 102. FIG. 6F is a perspective view of an example of the separated polymeric covering 177 from the cylinder 102 after performing the stages of the method in FIGS. 6A through 6E. In one example, a robot may be used to remove the separated polymeric covering 177 from the cylinder 102. In some examples, the robot is different than the robot 101 that was used to form the line of weakness and rupture the polymeric covering 106. In one example, the robot may feature a vacuum that is used to remove the separated covering 177 from being around the cylinder 102, so that the cylinder 102 can then be moved to the absorbent article manufacturing line. In yet another example, a robot may be used to lift the rolls 104 of the cylinder 102 to fully separate the rolls 104 from the separated covering 177 after which a vacuum or other similar component is used to remove the polymeric covering 177 from a vicinity of the rolls 104.

After the polymeric covering 106 is removed from the rolls 104 of the cylinder 102, the one or more rolls 104 may be transported to the absorbent article manufacturing line. In an example, the fibrous material of the one or more rolls 104 may then be automatically unwound at the absorbent article manufacturing line. In one example, an automatic means (e.g. vacuum device) may be used to automatically pick up the outer layer 134 (FIG. 5B) of the roll 104 and commence to unwind the layers of the roll 104. As appreciated by one skilled in the art, in some examples the absorbent article manufacturing method may remove a plurality of outer layers from each roll prior to using the roll layers in the manufacturing of the disposable absorbent article.

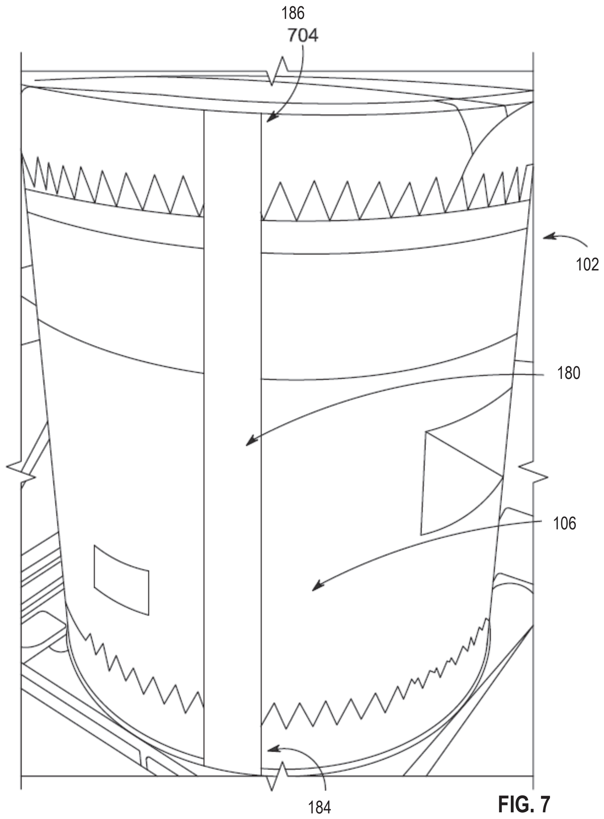

Referring to FIG. 7, typically, rolls of fibrous material may be shipped from the roll goods manufacturer to absorbent article manufactures in the cylinder 102 of 2-20 rolls, for example. The cylinders 102 may be about 1 meter to 2 meters in diameter, for example. The rolls 104 may be tightly wrapped with a plastic film, such as the polymeric covering 106 or a polyethylene film, to protect the rolls from dirt and damage. Cardboard may cover the cylinder 102 of the rolls 104 on the flat ends of the cylinder 102 to protect the ends. This plastic polymeric covering 106 may need to be removed prior to using the rolls 104 in absorbent article manufacturing process. Typically, the polymeric covering 106 may be manually cut off using a rotary sheer or cutter, however when cutting across the polymeric covering 106 over the curved outer surface of the rolls the manual rotary cutting may damage and cut into the outer revolutions of the fibrous material on the rolls, thereby ruining the outer revolutions of the fibrous material. It was discovered that by adding a sacrificial material 180 in a strip about the curved outer surface of the cylinder 102 from the first end 184 to the second end 186 and under the polymeric covering 106, automated cutting may occur to remove the polymeric covering 106 without damaging the outer layers 134, 135 of the rolls 104. In such a way, a robot 101 may use a rotary sheer or cutter to cut into but not through the sacrificial material 180 while also cutting the polymeric covering 106. Examples of the sacrificial material are heavy paper, cardboard, corrugated cardboard, polymer foam, and/or other low cost materials that may provide sufficient caliper for protection of the underlying roll layers. The sacrificial material may comprise a single layer or may be a laminate of multiple materials. The strip of sacrificial material may be from about 0.5 inches wide to about 15 inches wide, for example. This automated process may significantly reduce scrap caused by the manual cutting and damage to the outer layers 134, 135 of the roll 104 material.

FIG. 8 is a flowchart depicting an example of one or more steps of a method 200 for automatically removing a polymeric covering 106 from a cylinder 102 including one or more roll 104 of fibrous material Although the flow diagram of FIG. 8 may be depicted as integral steps in a particular order for purposes of illustration, in other embodiments one or more steps, or portions thereof, are performed in a different order, or overlapping in time, in series or in parallel, or are deleted, or one or more other steps may be added, or the method may be changed in some combination of ways.

In an example, the disposable absorbent article is a pant, a taped diaper, or a sanitary napkin.

In step 202, the cylinder 102 may be provided with one or more rolls 104a, 104b of fibrous material. As previously discussed, the cylinder 102 provided in step 202 may be wrapped with the polymeric covering 106, such as over the top 158 of the cylinder 102 and along first and second sides 154, 164, as well as along a bottom (not shown) of the cylinder 102.

In step 204, the zone of weakness may be formed in the axial direction 112 of the cylinder 102 in the polymeric covering 106 with the robot 101. In one example, in step 204 the zone of weakness may also be formed along one or more of the first side 154 of the cylinder 102 (FIG. 6A); over the first corner 170 of the cylinder 102 (FIG. 6B); over the top 158 of the cylinder 102 (FIG. 6C); over the second corner 176 of the cylinder 102 (FIG. 6D) and/or along the second side 164 of the cylinder 102 (FIG. 6E). In an example, the zone of weakness may be the line of weakness 110 and may be formed by the spokes 122 of the projection wheel 120'.

In step 206, the breaking device 128 may be provided with a temperature that is within a certain range (e.g. about 30 degrees Celsius) of the melting temperature of the polymeric covering 106 and/or at or above the melting temperature of the polymeric covering 106. In one example, the heating device 119 may be activated by the controller 114 in step 206 until the breaking device 128 is at a desired temperature.

In step 208, the robot 101 may be used to apply a substantially uniform radially inward force against the cylinder 102 with the breaking device 128 along the zone of weakness formed in step 206, so to rupture the zone of weakness. In one example, in step 208 the force control device 130 may ensure that the breaking device 128 does not impart a force on the cylinder 102 (e.g. including the polymeric covering 106 and one or more outer layers 134, 135 of rolls 104 of the cylinder 102) that exceeds a maximum threshold force. It was recognized that this may advantageously ensure that no two adjacent outer layers 134, 135 of a roll 104 are fused together over a fused width 190 (FIG. 5B) that exceeds a certain ratio (e.g. about 20%) of the roll width 189. Consequently, it was recognized that step 208 may advantageously ensure that once the polymeric covering 106 is removed, the rolls 104 can be used in a subsequent step of the absorbent article manufacturing method such as the automatic feed step where the outer layer 134 of a roll 104 is automatically picked up by a robot (e.g. using a vacuum means).

In another example, in step 208 the applying step may involve biasing the breaking device 128 against the cylinder 102 using the force control device 130, such that an imparted force of the breaking device 128 on the cylinder 102 does not exceed a maximum threshold force. In an example , the maximum threshold force is 25 N. In another example, the maximum threshold force may depend on how much heat is transferred as a function of one or more of a starting temperature, a tool speed, a number of polymeric cover layers and/or a melting temperature of the non-woven material.

In step 210, a robot may be used to remove the polymeric covering 106 from the one or more rolls 104, after the zones of weakness were ruptured in step 208. Thus, in step 210 the robot may just remove the polymeric covering 106 from a vicinity of the rolls 104, after the polymeric covering 106 was removed in step 208 from its packaging of the cylinder 102.

In step 212, after the polymeric covering 106 is removed from the cylinder 102 and the rolls 104 within the cylinder 102 are transported to the absorbent article manufacturing line, one or more subsequent steps of the absorbent article manufacturing method may be performed. In one example, these steps may include an automatic unwinding of the layers 134, 135 of the rolls 104, such as using a robot to automatically pick up the outer most layer 134 of a roll 104 and commencing to unwind the fibrous material of the roll 104. In an example, as previously discussed, since the improved method may ensure that a fused width 190 over which multiple outer layers are melted or fused together does not exceed a certain ratio (e.g. about 10% to about 30%) of the roll width 189, it was recognized that the fibrous material of these rolls may be unwound in step 212 without any interference due to damage to the outer layers of the roll.

In some examples, after step 212, the absorbent article manufacturing method may discard a certain number of the outer layers of the roll 104 after unwinding these outer layers from the roll 104, in order to minimize the risk of contamination to the method of forming the absorbent articles.

### Aspects

The disclosure herein may feature one or more aspects , such as but not limited to:
A. A method of manufacturing a disposable absorbent article comprising:
   providing a cylinder of at least one roll of fibrous material, wherein the cylinder of the at least one roll of fibrous material is at least partially wrapped with a polymeric covering having a tension in the circumferential direction of the cylinder;
   forming a zone of weakness in an axial direction of the cylinder in the polymeric covering using a robot, wherein the zone of weakness comprises land areas and open areas;
   providing a breaking device at a temperature within 150 degrees Celsius of the melting temperature of the polymeric covering;
   wherein the fibrous material has a melting temperature above the melting temperature of the polymeric covering by at least 10 degrees Celsius;
   using the robot, applying a substantially uniform radially inward force against the cylinder with the breaking device along the zone of weakness to rupture the zone of weakness;
   during the applying step, not damaging outer layers of the fibrous material;
   using a robot to remove the polymeric covering from the at least one roll; and
   unwinding the at least one roll and feeding the at least one roll into an absorbent article manufacturing line.
B. The aspect of paragraph A, wherein the forming the zone of weakness step comprises forming one or more lines of weakness in the axial direction and wherein the applying step is performed in a direction that is parallel with the one or more lines of weakness in the axial direction.
C. The aspect of paragraph B, wherein the forming the one or more lines of weakness step comprises forming a single line of weakness in the axial direction and wherein the applying step is performed in the direction that is aligned with the single line of weakness in the axial direction.
D. The aspect of paragraph B, wherein the forming the one or more lines of weakness step comprises forming a pair of parallel lines of weakness in the axial direction and wherein the applying step is performed in the direction between the pair of parallel lines of weakness in the axial direction.
E. The aspect of paragraph B, wherein the forming the one or more lines of weakness comprises perforating the polymeric covering to form the land areas and the open areas.
F. The aspect of any of the preceding paragraphs, wherein the fibrous material comprises one or more of a nonwoven material, polyfilms, and laminates.
G. The aspect of any one of the preceding paragraphs, wherein the breaking device is a severing device.
H. The aspect of any one of the preceding paragraphs, wherein the breaking device is a melting device.
I. The aspect of any one of the preceding paragraphs, wherein the polymeric covering comprises more than one layer.
J. The aspect of any one of the preceding paragraphs, comprising biasing the breaking device toward the zone of weakness to account for variations in the cylinder along the zone of weakness.
K. The aspect of any one of the preceding paragraphs, wherein the at least one roll comprises two or more individual rolls of the fibrous material.
L. The aspect of any one of the preceding paragraphs, wherein the absorbent article is a pant, a taped diaper, or a sanitary napkin.
M. The aspect of any one of the preceding paragraphs, wherein the breaking device comprises a first end with a pointed projection and a second end spaced a distance apart, wherein at the first end of the breaking device is shorter, and wherein at the second end of the breaking device is longer.
N. The aspect of any one of the preceding paragraphs, wherein the rupturing the zone of weakness occurs at a rate of about 3 inches to about 8 inches per second.
O. The aspect of any one of the preceding paragraphs, wherein for each of the at least one roll in the cylinder, the applying step comprises melting not more than a maximum percentage of a width of an outer layer of the roll to an inner layer of the roll such that the unwinding step is not affected by the melting, wherein the maximum ratio is between about 10% and about 30%.
P. The aspect of any one of the preceding paragraphs, wherein the temperature of the breaking device is at or above the melting temperature of the polymeric covering.
Q. The aspect of any one of the preceding paragraphs, wherein the forming step comprises increasing the tension in the polymeric covering in the circumferential direction per unit length along the zone of weakness; and
   wherein the substantially uniform radially inward force is at least a threshold minimum force to rupture the zone of weakness and wherein the threshold minimum force is reduced based on the increased tension per unit length along the zone of weakness.
R. The aspect of any one of the preceding paragraphs, wherein the applying step comprises selecting a value of at least one of the temperature of the breaking device and a speed of the breaking device along the zone of weakness, and wherein the selecting step is performed such that a thermal transfer rate from the breaking device to the at least one roll of the cylinder is not greater than a maximum threshold rate to prevent a melting or fusing together of more than one outer layer of the roll.
S. The aspect of any one of the preceding paragraphs, wherein the forming step is performed by a perforation wheel comprising a plurality of spokes around the circumference of the perforation wheel, and wherein the forming step comprises rotating the perforation wheel such that the plurality of spokes form a plurality of perforations in the polymeric covering to form the land areas and the open areas along the zone of weakness.
T. The aspect of paragraph S, wherein the breaking device is a wheel with the pointed projection at the first end along an outer circumference of the wheel and the second end is positioned radially inward of the outer circumference.
U. The aspect of any one of the preceding paragraphs, wherein after the unwinding step the method further comprises removing and discarding a plurality of outer layers of the at least one roll prior to the absorbent article manufacturing line using the at least one roll to form an absorbent article.
V. The aspect of any one of the preceding paragraphs, wherein the applying step comprises:
   automatically moving, with the robot, the breaking device in a first path along a first radial direction along a first side of the cylinder to rupture the zone of weakness in the polymeric covering along the first side of the cylinder, wherein the first radial direction is perpendicular to the axial direction;
   automatically moving, with the robot, the breaking device in a second path along the axial direction along a top of the cylinder to rupture the zone of weakness in the polymeric covering along the top of the cylinder; and
   automatically moving, with the robot, the breaking device in a third path along a second radial direction opposite to the first radial direction along a second side of the cylinder to rupture the zone of weakness in the polymeric coating along the second side of the cylinder;
   wherein the first path, the second path and the third path form a single plane.
W. The aspect of paragraph V, further comprising:
   automatically moving, with the robot, the breaking device in a fourth path in a mixed direction including the first radial direction and the axial direction over a first corner of the cylinder between the first side and the top side to rupture the zone of weakness in the polymeric covering along the first corner; and
   automatically moving, with the robot, the breaking device in a fifth path in a mixed direction including the second radial direction and the axial direction over a second corner of the cylinder between the top side and the second side to rupture the zone of weakness in the polymeric coating along the second corner.
X. The aspect of paragraph V, wherein the automatically moving steps further comprise automatically controlling, with the robot, a position of the breaking device in each of the automatically moving steps such that the position of the breaking device remains in the single plane.
Y. The aspect of paragraph L, wherein the biasing step comprises imparting, with a force control device, a force with the breaking device on the cylinder due to the variations in the cylinder including variations in an outer diameter of the cylinder along the axial direction, and wherein the imparted force does not exceed a maximum threshold force during the applying step.
Z. The aspect of paragraph Y, wherein the biasing step comprises:
   imparting, with a first force control device, a force with the breaking device on the cylinder due to the variations in the cylinder including variations in the outer diameter of the cylinder; and
   imparting, with a second force control device, a force with a perforation wheel comprising a plurality of spokes on the cylinder due to the variations in the cylinder including variations in the outer diameter of the cylinder, wherein the forming step is performed by the perforation wheel.

AA. The aspect of paragraph Z, wherein the first force control device comprises robot joints and wherein the second force control device comprises a spring.
AB. The aspect of paragraph Y, wherein the breaking device is spring loaded such that the force control device is a spring having a value of a spring constant, wherein a value of the maximum threshold force is based on the value of the spring constant, and wherein the method further comprises selecting the value of the spring constant such that the maximum threshold force is not greater than about 25 N .
AC. The aspect of paragraph Y, wherein the force control device is a pneumatic air cylinder.

### Hardware

FIG. 9 may be a block diagram that illustrates a computer system 300 upon which an embodiment of the invention may be implemented. Computer system 300 includes a communication mechanism such as a bus 310 for passing information between other internal and external components of the computer system 300. Information may be represented as physical signals of a measurable phenomenon, typically electric voltages, but including, in other embodiments, such phenomena as magnetic, electromagnetic, pressure, chemical, molecular atomic and quantum interactions. For example, north and south magnetic fields, or a zero and non-zero electric voltage, represent two states (0, 1) of a binary digit (bit). Other phenomena may represent digits of a higher base. A superposition of multiple simultaneous quantum states before measurement represents a quantum bit (qubit). A sequence of one or more digits constitutes digital data that may be used to represent a number or code for a character. In some embodiments, information called analog data may be represented by a near continuum of measurable values within a particular range. Computer system 300, or a portion thereof, constitutes a means for performing one or more steps of one or more methods described herein.

A sequence of binary digits constitutes digital data that may be used to represent a number or code for a character. A bus 310 includes many parallel conductors of information so that information may be transferred quickly among devices coupled to the bus 310. One or more processors 302 for processing information are coupled with the bus 310. A processor 302 performs a set of operations on information. The set of operations include bringing information in from the bus 310 and placing information on the bus 310. The set of operations also typically include comparing two or more units of information, shifting positions of units of information, and combining two or more units of information, such as by addition or multiplication. A sequence of operations to be executed by the processor 302 constitutes computer instructions.

Computer system 300 also includes a memory 304 coupled to bus 310. The memory 304, such as a random access memory (RAM) or other dynamic storage device, stores information including computer instructions. Dynamic memory allows information stored therein to be changed by the computer system 300. RAM allows a unit of information stored at a location called a memory address to be stored and retrieved independently of information at neighboring addresses. The memory 304 may be also used by the processor 302 to store temporary values during execution of computer instructions. The computer system 300 also includes a read only memory (ROM) 306 or other static storage device coupled to the bus 310 for storing static information, including instructions, that may not be changed by the computer system 300. Also coupled to bus 310 may be a non-volatile (persistent) storage device 308, such as a magnetic disk or optical disk, for storing information, including instructions, that persists even when the computer system 300 may be turned off or otherwise loses power.

Information, including instructions, may be provided to the bus 310 for use by the processor from an external input device 312, such as a keyboard containing alphanumeric keys operated by a human user, or a sensor. A sensor detects conditions in its vicinity and transforms those detections into signals compatible with the signals used to represent information in computer system 300. Other external devices coupled to bus 310, used primarily for interacting with humans, include a display device 314, such as a cathode ray tube (CRT) or a liquid crystal display (LCD), for presenting images, and a pointing device 316, such as a mouse or a trackball or cursor direction keys, for controlling a position of a small cursor image presented on the display 314 and issuing commands associated with graphical elements presented on the display 314.

In the illustrated embodiment, special purpose hardware, such as an application specific integrated circuit (IC) 320, may be coupled to bus 310. The special purpose hardware may be configured to perform operations not performed by processor 302 quickly enough for special purposes. Examples of application specific ICs include graphics accelerator cards for generating images for display 314, cryptographic boards for encrypting and decrypting messages sent over a network, speech recognition, and interfaces to special external devices, such as robotic arms and medical scanning equipment that repeatedly perform some complex sequence of operations that are more efficiently implemented in hardware.

Computer system 300 also includes one or more instances of a communications interface 370 coupled to bus 310. Communication interface 370 provides a two-way communication coupling to a variety of external devices that operate with their own processors, such as printers, scanners and external disks. In general, the coupling may be with a network link 378 that may be connected to a local network 380 to which a variety of external devices with their own processors are connected. For example, communication interface 370 may be a parallel port or a serial port or a universal serial bus (USB) port on a personal computer. In some embodiments, communications interface 370 may be an integrated services digital network (ISDN) card or a digital subscriber line (DSL) card or a telephone modem that provides an information communication connection to a corresponding type of telephone line. In some embodiments, a communication interface 370 may be a cable modem that converts signals on bus 310 into signals for a communication connection over a coaxial cable or into optical signals for a communication connection over a fiber optic cable. As another example, communications interface 370 may be a local area network (LAN) card to provide a data communication connection to a compatible LAN, such as Ethernet. Wireless links may also be implemented. Carrier waves, such as acoustic waves and electromagnetic waves, including radio, optical and infrared waves travel through space without wires or cables. Signals include man-made variations in amplitude, frequency, phase, polarization or other physical properties of carrier waves. For wireless links, the communications interface 370 sends and receives electrical, acoustic or electromagnetic signals, including infrared and optical signals, that carry information streams, such as digital data.

The term computer-readable medium may be used herein to refer to any medium that participates in providing information to processor 302, including instructions for execution. Such a medium may take many forms, including, but not limited to, non-volatile media, volatile media and transmission media. Non-volatile media include, for example, optical or magnetic disks, such as storage device 308. Volatile media include, for example, dynamic memory 304. Transmission media include, for example, coaxial cables, copper wire, fiber optic cables, and waves that travel through space without wires or cables, such as acoustic waves and electromagnetic waves, including radio, optical and infrared waves. The term computer-readable storage medium may be used herein to refer to any medium that participates in providing information to processor 302, except for transmission media.

Common forms of computer-readable media include, for example, a floppy disk, a flexible disk, a hard disk, a magnetic tape, or any other magnetic medium, a compact disk ROM (CD-ROM), a digital video disk (DVD) or any other optical medium, punch cards, paper tape, or any other physical medium with patterns of holes, a RAM, a programmable ROM (PROM), an erasable PROM (EPROM), a FLASH-EPROM, or any other memory chip or cartridge, a carrier wave, or any other medium from which a computer may read. The term non-transitory computer-readable storage medium may be used herein to refer to any medium that participates in providing information to processor 302, except for carrier waves and other signals.

Logic encoded in one or more tangible media includes one or both of processor instructions on a computer-readable storage media and special purpose hardware, such as ASIC *320.

Network link 378 typically provides information communication through one or more networks to other devices that use or process the information. For example, network link 378 may provide a connection through local network 380 to a host computer 382 or to equipment 384 operated by an Internet Service Provider (ISP). ISP equipment 384 in turn provides data communication services through the public, world-wide packet-switching communication network of networks now commonly referred to as the Internet 390. A computer called a server 392 connected to the Internet provides a service in response to information received over the Internet. For example, server 392 provides information representing video data for presentation at display 314.

The disclosure may be related to the use of computer system 300 for implementing the techniques described herein. According to one embodiment of the invention, those techniques are performed by computer system 300 in response to processor 302 executing one or more sequences of one or more instructions contained in memory 304. Such instructions, also called software and program code, may be read into memory 304 from another computer-readable medium such as storage device 308. Execution of the sequences of instructions contained in memory 304 causes processor 302 to perform the method steps described herein. In alternative embodiments, hardware, such as application specific integrated circuit 320, may be used in place of or in combination with software to implement the invention. Thus, embodiments of the invention are not limited to any specific combination of hardware and software.

The signals transmitted over network link 378 and other networks through communications interface 370, carry information to and from computer system 300. Computer system 300 may send and receive information, including program code, through the networks 380, 390 among others, through network link 378 and communications interface 370. In an example using the Internet 390, a server 392 transmits program code for a particular application, requested by a message sent from computer 300, through Internet 390, ISP equipment 384, local network 380 and communications interface 370. The received code may be executed by processor 302 as it may be received, or may be stored in storage device 308 or other non-volatile storage for later execution, or both. In this manner, computer system 300 may obtain application program code in the form of a signal on a carrier wave.

Various forms of computer readable media may be involved in carrying one or more sequence of instructions or data or both to processor 302 for execution. For example, instructions and data may initially be carried on a magnetic disk of a remote computer such as host 382. The remote computer loads the instructions and data into its dynamic memory and sends the instructions and data over a telephone line using a modem. A modem local to the computer system 300 receives the instructions and data on a telephone line and uses an infra-red transmitter to convert the instructions and data to a signal on an infra-red a carrier wave serving as the network link 378. An infrared detector serving as communications interface 370 receives the instructions and data carried in the infrared signal and places information representing the instructions and data onto bus 310. Bus 310 carries the information to memory 304 from which processor 302 retrieves and executes the instructions using some of the data sent with the instructions. The instructions and data received in memory 304 may optionally be stored on storage device 308, either before or after execution by the processor 302.

FIG. 10 illustrates a chip set 400 upon which an embodiment of the invention may be implemented. Chip set 400 may be programmed to perform one or more steps of a method described herein and includes, for instance, the processor and memory components described with respect to FIG. 1 incorporated in one or more physical packages (e.g., chips). By way of example, a physical package includes an arrangement of one or more materials, components, and/or wires on a structural assembly (e.g., a baseboard) to provide one or more characteristics such as physical strength, conservation of size, and/or limitation of electrical interaction. It may be contemplated that in certain embodiments the chip set may be implemented in a single chip. Chip set 400, or a portion thereof, constitutes a means for performing one or more steps of a method described herein.

In one embodiment, the chip set 400 includes a communication mechanism such as a bus 401 for passing information among the components of the chip set 400. A processor 403 has connectivity to the bus 401 to execute instructions and process information stored in, for example, a memory 405. The processor 403 may include one or more processing cores with each core configured to perform independently. A multi-core processor enables multiprocessing within a single physical package. Examples of a multi-core processor include two, four, eight, or greater numbers of processing cores. Alternatively or in addition, the processor 403 may include one or more microprocessors configured in tandem via the bus 401 to enable independent execution of instructions, pipelining, and multithreading. The processor 403 may also be accompanied with one or more specialized components to perform certain processing functions and tasks such as one or more digital signal processors (DSP) 407, or one or more application-specific integrated circuits (ASIC) 409. A DSP 407 typically may be configured to process real-world signals (e.g., sound) in real time independently of the processor 403. Similarly, an ASIC 409 may be configured to performed specialized functions not easily performed by a general purposed processor. Other specialized components to aid in performing the inventive functions described herein include one or more field programmable gate arrays (FPGA) (not shown), one or more controllers (not shown), or one or more other special-purpose computer chips.

The processor 403 and accompanying components have connectivity to the memory 405 via the bus 401. The memory 405 includes both dynamic memory (e.g., RAM, magnetic disk, writable optical disk, etc.) and static memory (e.g., ROM, CD-ROM, etc.) for storing executable instructions that when executed perform one or more steps of a method described herein. The memory 405 also stores the data associated with or generated by the execution of one or more steps of the methods described herein.

FIG. 11 may be a diagram of exemplary components of a mobile terminal 500 (e.g., cell phone handset) for communications, which may be capable of operating in the system of FIG. 1, according to one embodiment. In some embodiments, mobile terminal 501, or a portion thereof, constitutes a means for performing one or more steps described herein. Generally, a radio receiver may be often defined in terms of front-end and back-end characteristics. The front-end of the receiver encompasses all of the Radio Frequency (RF) circuitry whereas the back-end encompasses all of the base-band processing circuitry. As used in this application, the term "circuitry" refers to both: (1) hardware-only implementations (such as implementations in only analog and/or digital circuitry), and (2) to combinations of circuitry and software (and/or firmware) (such as, if applicable to the particular context, to a combination of processor(s), including digital signal processor(s), software, and memory(ies) that work together to cause an apparatus, such as a mobile phone or server, to perform various functions). This definition of "circuitry" applies to all uses of this term in this application, including in any claims. As a further example, as used in this application and if applicable to the particular context, the term "circuitry" would also cover an implementation of merely a processor (or multiple processors) and its (or their) accompanying software/or firmware. The term "circuitry" would also cover if applicable to the particular context, for example, a baseband integrated circuit or applications processor integrated circuit in a mobile phone or a similar integrated circuit in a cellular network device or other network devices.

Pertinent internal components of the telephone include a Main Control Unit (MCU) 503, a Digital Signal Processor (DSP) 505, and a receiver/transmitter unit including a microphone gain control unit and a speaker gain control unit. A main display unit 507 provides a display to the user in support of various applications and mobile terminal functions that perform or support the steps as described herein. The display 507 includes display circuitry configured to display at least a portion of a user interface of the mobile terminal (e.g., mobile telephone). Additionally, the display 507 and display circuitry are configured to facilitate user control of at least some functions of the mobile terminal. An audio function circuitry 509 includes a microphone 511 and microphone amplifier that amplifies the speech signal output from the microphone 511. The amplified speech signal output from the microphone 511 may be fed to a coder/decoder (CODEC) 513.

A radio section 515 amplifies power and converts frequency in order to communicate with a base station, which may be included in a mobile communication system, via antenna 517. The power amplifier (PA) 519 and the transmitter/modulation circuitry are operationally responsive to the MCU 503, with an output from the PA 519 coupled to the duplexer 521 or circulator or antenna switch, as known in the art. The PA 519 also couples to a battery interface and power control unit 520.

In use, a user of mobile terminal 501 speaks into the microphone 511 and his or her voice along with any detected background noise may be converted into an analog voltage. The analog voltage may be then converted into a digital signal through the Analog to Digital Converter (ADC) 523. The control unit 503 routes the digital signal into the DSP 505 for processing therein, such as speech encoding, channel encoding, encrypting, and interleaving. In one embodiment, the processed voice signals are encoded, by units not separately shown, using a cellular transmission protocol such as enhanced data rates for global evolution (EDGE), general packet radio service (GPRS), global system for mobile communications (GSM), Internet protocol multimedia subsystem (IMS), universal mobile telecommunications system (UMTS), etc., as well as any other suitable wireless medium, e.g., microwave access (WiMAX), Long Term Evolution (LTE) networks, code division multiple access (CDMA), wideband code division multiple access (WCDMA), wireless fidelity (WiFi), satellite, and the like, or any combination thereof.

The encoded signals are then routed to an equalizer 525 for compensation of any frequency-dependent impairments that occur during transmission though the air such as phase and amplitude distortion. After equalizing the bit stream, the modulator 527 combines the signal with a RF signal generated in the RF interface 529. The modulator 527 generates a sine wave by way of frequency or phase modulation. In order to prepare the signal for transmission, an up-converter 531 combines the sine wave output from the modulator 527 with another sine wave generated by a synthesizer 533 to achieve the desired frequency of transmission. The signal may be then sent through a PA 519 to increase the signal to an appropriate power level. In practical systems, the PA 519 acts as a variable gain amplifier whose gain may be controlled by the DSP 505 from information received from a network base station. The signal may be then filtered within the duplexer 521 and optionally sent to an antenna coupler 535 to match impedances to provide maximum power transfer. Finally, the signal may be transmitted via antenna 517 to a local base station. An automatic gain control (AGC) may be supplied to control the gain of the final stages of the receiver. The signals may be forwarded from there to a remote telephone which may be another cellular telephone, any other mobile phone or a land-line connected to a Public Switched Telephone Network (PSTN), or other telephony networks.

Voice signals transmitted to the mobile terminal 501 are received via antenna 517 and immediately amplified by a low noise amplifier (LNA) 537. A down-converter 539 lowers the carrier frequency while the demodulator 541 strips away the RF leaving only a digital bit stream. The signal then goes through the equalizer 525 and may be processed by the DSP 505. A Digital to Analog Converter (DAC) 543 converts the signal and the resulting output may be transmitted to the user through the speaker 545, all under control of a Main Control Unit (MCU) 503 which may be implemented as a Central Processing Unit (CPU) (not shown).

The MCU 503 receives various signals including input signals from the keyboard 547. The keyboard 547 and/or the MCU 503 in combination with other user input components (e.g., the microphone 511) comprise a user interface circuitry for managing user input. The MCU 503 runs a user interface software to facilitate user control of at least some functions of the mobile terminal 501 as described herein. The MCU 503 also delivers a display command and a switch command to the display 507 and to the speech output switching controller, respectively. Further, the MCU 503 exchanges information with the DSP 505 and may access an optionally incorporated SIM card 549 and a memory 551. In addition, the MCU 503 executes various control functions required of the terminal. The DSP 505 may, depending upon the implementation, perform any of a variety of conventional digital processing functions on the voice signals. Additionally, DSP 505 determines the background noise level of the local environment from the signals detected by microphone 511 and sets the gain of microphone 511 to a level selected to compensate for the natural tendency of the user of the mobile terminal 501.

The CODEC 513 includes the ADC 523 and DAC 543. The memory 551 stores various data including call incoming tone data and may be capable of storing other data including music data received via, e.g., the global Internet. The software module could reside in RAM memory, flash memory, registers, or any other form of writable storage medium known in the art. The memory device 551 may be, but not limited to, a single memory, CD, DVD, ROM, RAM, EEPROM, optical storage, magnetic disk storage, flash memory storage, or any other non-volatile storage medium capable of storing digital data.

An optionally incorporated SIM card 549 carries, for instance, important information, such as the cellular phone number, the carrier supplying service, subscription details, and security information. The SIM card 549 serves primarily to identify the mobile terminal 501 on a radio network. The card 549 also contains a memory for storing a personal telephone number registry, text messages, and user specific mobile terminal settings.

In some embodiments, the mobile terminal 501 includes a digital camera comprising an array of optical detectors, such as charge coupled device (CCD) array 565. The output of the array may be image data that may be transferred to the MCU for further processing or storage in the memory 551 or both. In the illustrated embodiment, the light impinges on the optical array through a lens 563, such as a pin-hole lens or a material lens made of an optical grade glass or plastic material. In the illustrated embodiment, the mobile terminal 501 includes a light source 561, such as a LED to illuminate a subject for capture by the optical array, e.g., CCD 565. The light source may be powered by the battery interface and power control module 520 and controlled by the MCU 503 based on instructions stored or loaded into the MCU 503.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present disclosure have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A method of manufacturing a disposable absorbent article comprising:
providing a cylinder (102) of at least one roll (104) of fibrous material, wherein the cylinder of the at least one roll of fibrous material is at least partially wrapped with a polymeric covering (106) having a tension in the circumferential direction of the cylinder;
forming a zone of weakness in an axial direction (112) of the cylinder in the polymeric covering (106) using a robot (101), wherein the zone of weakness comprises land areas (124) and open areas (126);
providing a breaking device (128) at a temperature within 150 degrees Celsius of the melting temperature of the polymeric covering;
wherein the fibrous material has a melting temperature above the melting temperature of the polymeric covering (106) by at least 10 degrees Celsius;
using the robot (101), applying a substantially uniform radially inward force against the cylinder (102) with the breaking device (128) along the zone of weakness to rupture the zone of weakness;
during the applying step, not damaging outer layers (134, 135) of the fibrous material;
using a robot to remove the polymeric covering (106) from the at least one roll (104); and
unwinding the at least one roll (104) and feeding the at least one roll (104) into an absorbent article manufacturing line.

2. The method of claim 1, wherein the forming the zone of weakness step comprises forming one or more lines of weakness (110) in the axial direction (112) and wherein the applying step is performed in a direction that is parallel with the one or more lines of weakness (110) in the axial direction (112).

3. The method of claim 2, wherein the forming the one or more lines of weakness step comprises perforating the polymeric covering (106) to form the land areas (124) and the open areas (126).

4. The method of any one of the preceding claims, wherein the fibrous material comprises one or more of a nonwoven material, polyfilms, and laminates.

5. The method of any one of the preceding claims, wherein the breaking device (128) is a severing device or a melting device.

6. The method of any one of the preceding claims, wherein the polymeric covering (106) comprises more than one layer.

7. The method of any one of the preceding claims, comprising biasing the breaking device (128) toward the zone of weakness to account for variations in the cylinder (102) along the zone of weakness.

8. The method of any one of the preceding claims, wherein the absorbent article is a pant, a taped diaper, or a sanitary napkin.

9. The method of any one of the preceding claims, wherein the breaking device (128) comprises a first end (144) with a pointed projection (140) and a second end (146) spaced a distance apart, wherein at the first end of the breaking device is shorter, and wherein at the second end of the breaking device is longer.

10. The method of any one of the preceding claims, wherein the rupturing the zone of weakness occurs at a rate of about 3 inches to about 8 inches per second.

11. The method of any one of the preceding claims, wherein for each of the at least one roll (104) in the cylinder (102), the applying step comprises melting not more than a maximum percentage of a width of an outer layer of the roll to an inner layer of the roll such that the unwinding step is not affected by the melting, wherein the maximum ratio is between about 10% and about 30%.

12. The method of any one of the preceding claims, wherein the temperature of the breaking device (128) is at or above the melting temperature of the polymeric covering.

13. The method of any one of the preceding claims, wherein the forming step comprises increasing the tension in the polymeric covering (106) in the circumferential direction per unit length along the zone of weakness; and
wherein the substantially uniform radially inward force is at least a threshold minimum force to rupture the zone of weakness and wherein the threshold minimum force is reduced based on the increased tension per unit length along the zone of weakness.

14. The method of any one of the preceding claims, wherein the applying step comprises selecting a value of at least one of the temperature of the breaking device (128) and a speed of the breaking device (128) along the zone of weakness, and wherein the selecting step is performed such that a thermal transfer rate from the breaking device (128) to the at least one roll (104) of the cylinder (102) is not greater than a maximum threshold rate to prevent a melting or fusing together of more than one outer layer of the roll.

15. The method of any one of the preceding claims, wherein the forming step is performed by a perforation wheel (120') comprising a plurality of spokes (122) around the circumference of the perforation wheel, and wherein the forming step comprises rotating the perforation wheel (120') such that the plurality of spokes (122) form a plurality of perforations in the polymeric covering to form the land areas (124) and the open areas (126) along the zone of weakness.
